(19) Europäisches Patentamt
European Patent Office
Office européen des brevets

(11) **EP 1 279 678 B1**

(12) **EUROPEAN PATENT SPECIFICATION**

(45) Date of publication and mention
of the grant of the patent:
**12.09.2007 Bulletin 2007/37**

(51) Int Cl.:
*C07K 14/16* (2006.01)   *C07K 16/10* (2006.01)
*C12Q 1/70* (2006.01)   *A61K 39/21* (2006.01)
*G01N 33/53* (2006.01)   *C12N 15/49* (2006.01)

(21) Application number: **02017001.5**

(22) Date of filing: **18.10.1985**

(54) **Gag antigen and its use for the detection of lav infection and in immunogenic compositions**

Gag-Antigen und dessen Verwendung zum Nachweis von LAV-Infektion, sowie in immunogenen Zusammensetzungen

Antigène gag et son utilisation pour la détection de l'infection avec LAV, et dans les composés immunogéniques

(84) Designated Contracting States:
**AT BE CH DE FR GB IT LI LU NL SE**

(30) Priority: **18.10.1984 FR 8416013**
**16.11.1984 GB 8429099**
**21.01.1985 GB 8501473**

(43) Date of publication of application:
**29.01.2003 Bulletin 2003/05**

(62) Document number(s) of the earlier application(s) in accordance with Art. 76 EPC:
**91113062.3 / 0 462 627**
**90105190.4 / 0 387 915**
**85905513.9 / 0 201 540**

(73) Proprietors:
- **INSTITUT PASTEUR**
  **75724 Paris Cédex 15 (FR)**
- **CENTRE NATIONAL DE LA RECHERCHE SCIENTIFIQUE**
  **75794 Paris Cedex 16 (FR)**

(72) Inventors:
- **Montagnier, Luc**
  **92350 Le Plessis-Robinson (FR)**
- **Krust, Bernard**
  **75012 Paris (FR)**
- **Chamaret, Solange**
  **75015 Paris (FR)**
- **Clavel, François**
  **75009 Paris (FR)**
- **Chermann, Jean-Claude**
  **13260 Cassis (FR)**
- **Barre-Sinoussi, Françoise**
  **92130 Issy-Les-Moulineaux (FR)**
- **Alizon, Marc**
  **75005 Paris (FR)**
- **Sonigo, Pierre**
  **75015 Paris (FR)**
- **Stewart, Cole**
  **92140 Clamart (FR)**
- **Danos, Olivier**
  **75015 Paris (FR)**
- **Wain-Hobson, Simon**
  **78180 Montigny-Le-Bretonneux (FR)**

(74) Representative: **Desaix, Anne et al**
**Ernest Gutmann - Yves Plasseraud S.A.S.**
**3, rue Auber**
**75009 Paris (FR)**

(56) References cited:
**EP-A- 0 178 978**

- **L. MONTAGNIER AT AL.: "Lymphadenopathy-associated virus and its etiological role in AIDS" RETROVIRUSES IN HUMAN LYMPHOMA/ LEUKEMIA, 1985, pages 319-331, XP008006450 & PRINCESS TAKAMATSU SYMPOSIUM, vol. 15, 1984, pages 319-331,**
- **V. S. KALYANARAM ET AL.: "Antibodies to the core protein of Lymphadenopathy-associated virus (LAV) in patients with AIDS" SCIENCE, vol. 225, 1984, pages 321-323, XP008006484**
- **F. BARR-SINOUSSI ET AL.: "Isolation of a T-lymphotropic retrovirus from a patient at risk for acquired immunodeficiency syndrome" SCIENCE, vol. 220, 1983, pages 868-870, XP008006485**

- **M. ALIZON ET AL.: "Molecular cloning of lymphadenopathy-associated virus" NATURE, vol. 312, 1984, pages 757-760, XP002208977**

**Description**

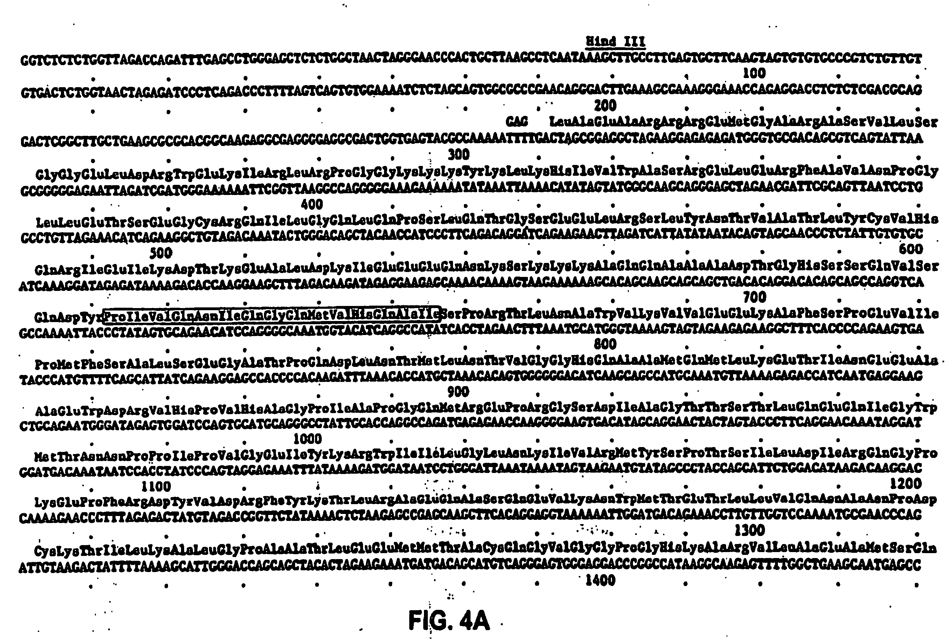

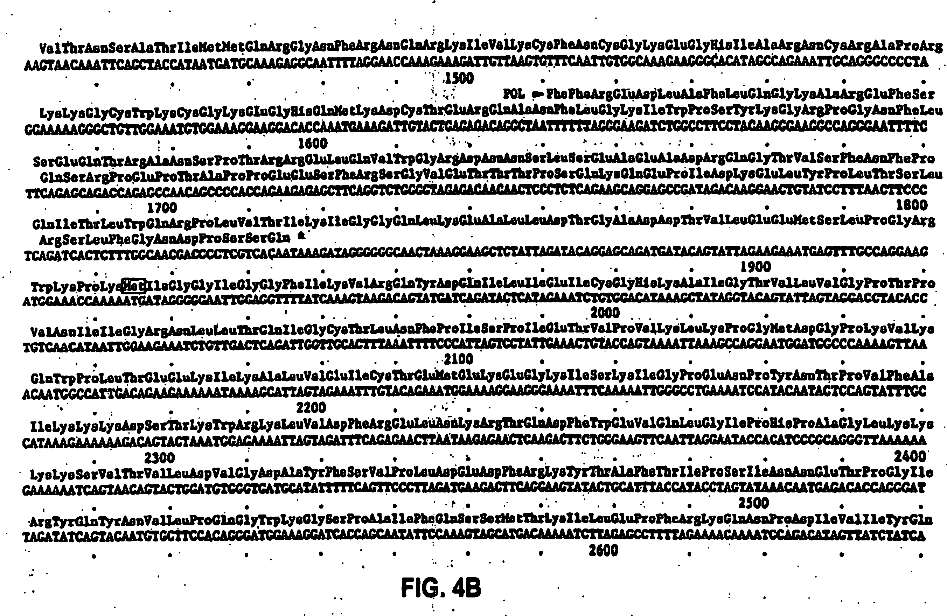

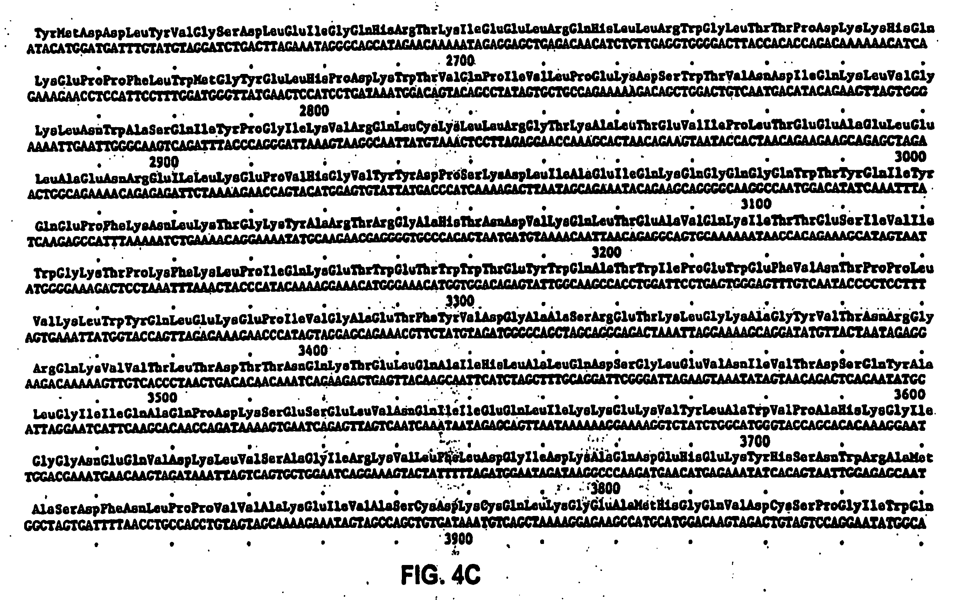

The present invention relates to antigens, particularly in a purified form, of the virus of lymphadenopathies (denoted below by the abbreviation LAS) and of the acquired immuno-depressive syndrome (denoted below by the abbreviation AIDS), to a process for producing these antigens, particularly recombinant or synthetic GAG antigen which consists in the aminoacid sequence represented on figure 4a and 4b beginning with residue Met at position 336 and ending with residue Gln at position 1836, or polypeptide fragments thereof of these viruses. The invention also relates to polypeptides, whether glycosylated or not, encoded by said DNA sequences.

The causative agent of LAS or AIDS, a retrovirus, has been identified by F. BARRE-SINOUSSI et al, Science, 220, 868 (1983). It has the following characteristics. It is T-lymphotropic; its prefered target is constituted by Leu 3 cells (or T4 lymphocytes) ; it has reverse transcriptase activity necessitating the presence of Mg + and exhibits strong affinity for poly(adenylate-oligodeoxy-thymidylate)(poly(A)-oligo(dT)12-18). It has a density of 1.16-1.17 in a sucrose gradient, an average diameter of 139 nanometers; and a nucleus having an average diameter of 41 nanometers. Antigens of said virus, particularly a protein p25 are recognised immunologically by antibodies contained in serums taken up from patients afflicted with LAS or AIDS. The p25 protein, which is a core protein, is not recognised immunologically by the p24 protein of the HTLVI and II viruses. The virus is also free of a p19 protein which is immunologically cross-reactive with the p19 proteins of HTLVI and HTLVII.

Retroviruses of this type (sometimes denoted by the generic abbreviation LAV) have been filed in the National Collection of Micro-organism Cultures of the INSTITUT PASTEUR of Paris, under numbers I-232, I-240 and I-241. Virus strains similar to LAV in all respects from the morphological and immunological point of view have been isolated in other laboratories. Reference is made by way of examples to the retrovirus strains named HTLV-III isolated by R.C. GALLO et al., Science, 224, 500 (1984) and by M.G. SARNGADHARAN et al., Science 224, 506 (1984) respectively and to the retrovirus isolated by M. JAY LEVY et al., Science, 225, 840-842 (1984), which virus was designated ARV. For the ease of language the last mentioned viruses, as well as others which have equivalent morphological and immunological properties, will be designated hereafter under the generic designation "LAV". Reference is also made to European patent application filed 14 September 1984, with the priority of British patent application number 83 24800 filed 15 september 1983 as regards a more detailed description of the LAV retroviruses or the like and of the uses to which extracts of these viruses give rise.

Initially the core antigens were the main antigens of the virus lysates or extracts which were recognised by serums of patients infected with AIDS or LAS, in the test systems which had then been used. A p42 protein, presented as consisting of an envelope protein, had been detected too. In the same manner GALLO et al disclosed a p41 protein which was also deemed to be on a possible component of the viru envelope.

Processes for obtaining a LAV virus have also been described. Reference may be made particularly to the article already mentioned of F. BARRE-SINOUSSI et al., as regards the preparation of the virus in T lymphocyte cultures derived either from blood, or from the umbilical cord, or also from bone marrow cells of adult donors in good health. This process comprises particularly the following essential steps :

-    producing a viral infection of these T lymphocytes, after activation by a lectin mitogen, with a viral suspension derived from a crude supernatant liquor of lymphocytes producing the virus (initially obtained from a patient infected with AIDS or LAS),
-    culturing cells infected with TCGF, in the presence of anti-α-interferon sheep serum,
-    effecting purification of the virus produced (production starts generally between the 9th and the 15th day following infection and lasts from 10 to 15 days), which purification comprises precipitating the virus in polyethylenglycol in order to produce a first concentration of the virus, then centrifugating the preparation obtained in a 20-60 % sucrose gradient or in an isotonic gradient of metrizanide (sold under the trade mark NYCODENZ by NYEGAARD, Oslo) and recovering the virus with the band having a density of 1.16-1.17 in the sucrose gradient or of 1.10-1.11 in the NYCODENZ® gradient.

The LAV virus may also be produced from permanent cell lines of type T, such as the CEM line, or from B lymphoblastoid cell lines, such as obtained by the transformation of the lymphocytes derived from a healthy donor with the Epstein-Barr virus, for instance as disclosed in French patent application Nr. 84 07151 filed May 9, 1984. The permanent cell lines obtained produce continuously a virus (designated as LAV-B in the case of the B lymphoblastoid cell lines) which possesses the essential antigenic and morphological features of the LAV viruses (except that it is collected in a density band sometimes slightly higher than in the preceding case (particularly 1.18) in sucrose. The final purification of the virus can also be carried out in a NYCODENZ®gradient.

Reference is made to the article of Schupbach et al. Science, vol. 224, pages 503-505. 4 May 1984. This article discloses a work with respect to different antigens of the HTLV-III retrovirus.

Results are presented including a reference to an antigen having migrated on an electrophoresis gel at a Migration distance corresponding to a molecular weight of approximately 110.000.

Schupbaen et al mentioned that this antigen was detected in a virus preparation but was below limit of detection in the cells.

A method for cloning DNA sequences hybridizable with the genomic RNA of LAS has already been disclosed in British Patent Application Nr. 84 23659 filed on September 19, 1984. Reference is hereafter made to that application as concerns subject matter in common with the further improvements to the invention disclosed herein.

The application describes providing purified unaltered virus forms (or viruses less altered by the purification procedures resorted to) and processes for obtaining said unaltered purified viruses.

The present invention further aims at providing additional new means which should not only also be useful for the detection of LAV or related viruses (hereafter more generally referred to as "LAV viruses"), but also have more versatility, particularly in detecting specific parts of the genomic DNA of said viruses whose expression products are not always directly detectable by immunological methods. The present invention further aims at providing polypeptides containing sequences in common with polypeptides comprising antigenic determinants included in the proteins encoded and expressed by the LAV genome occuring in nature. An additional object of the invention is to further provide means for the detection of proteins related to LAV virus, particularly for the diagnosis of AIDS or pre-AIDS or, to the contrary, for the detection of antibodies against the LAV virus or proteins related therewith, particularly in patients afflicted with AIDS or pre-AIDS or more generally in asymptomatic carriers and in blood-related products. Finally the invention also aims at providing immunogenic polypeptides, and more particularly protective polypeptides for use in the preparation of vaccine compositions against AIDS or related syndroms.

The present invention relates to additional DNA fragments, hybridizable with the genomic RNA of LAV as they will be disclosed hereafter, as well as with additional cDNA variants corresponding to the whole genomes of LAV viruses. It further relates to DNA recombinants containing said DNAs or cDNA fragments.

An unaltered purified LAV retrovirus distinguishes from those which have been defined above, in that it includes an amount of one or several envelope antigens, sufficient to be visualized when the virus is labelled with $^{35}$S-cystein, free of unlabelled cystein in a proportion of 200 microcuries per ml of medium, these antigens, among which particularly glycoproteins, are recognised selectively in vitro by serums of patients affected with SIDA or SLAs or by the serums of asymptomatic carriers of the virus asymptomatic carriers or the virus.

The application discloses antigen according to the preceding definition obtainable from a lysate of this virus (or by gentle scouring of the envelopes of the virus) is a glycoprotein having a molecular weight of the order of 110,000 daltons, as determined by its migration distance in comparison with the distances of migrations, in a same migration system, of standard proteins having known molecular weights. Particularly comparative measurements were made on a, 12.5 % polyacrylamid gel under a voltage of 18 V for 18 hours, upon using the following standard proteins (marketed by AMERSHAM) :

- lysozyme-($^{14}$C)-methyl (MW: 14, 300),
- carbon dioxide-($^{14}$C)-methyl (MW: 30,000),
- ovalbumin-($^{14}$C)-methyl (MW: 46,000),
- bovin albumin serum ($^{14}$C)-methyl (MW: 69,000),
- phosphorylase b-($^{14}$C)-methyl (MW: 92, 500),
- myosine-($^{14}$C)-methyl (MW: 200,000).

The application discloses also the antigens themselves, particularly that of molecular weight of about 110,000-120,000, which possess also the capability of being recognised by serums of patients infected with AIDS or LAS or by serums of persons who have been exposed to LAV viruses or those analogous with the latter. These antigens have also the characteristic of forming complexes with concanavaline A, said complex being dissociatable in the presence of O-methyl-$\alpha$-D-mannopyranoside. The antigens according to the invention can also bind to other lectins for example those known under the name "LENTYL-LECTIN". The preferred antigen according to the invention, of molecular weight 1100,000, is also sensitive to the action of endoglycosidases. This action is manifested by the production from the antigen of molecular weight 110,000 of a protein having a molecular weight of the order of 90,000, the latter being separable for example by immunoprecipitation or by separation employing the differences in molecular weights (migrations differentiated on gel).

The application discloses also the process for producing the viruses described herein. This process distinguishes essentially from those recalled herein above at the level of the final purification operation. In particular, the purification step of the process according to the application is no longer carried out in gradients, but involves the performance of differential centrifugations effected directly on the supernatants of the culture media of the producing cells. These centrifugation operations comprise particularly a first centrifugation at an angular centrifugation velocity, particularly of 10,000 rpm, enabling the removal of non-viral constituents, more particularly of cellular constituents, then a second centrifugation at higher angular velocity, particularly at 45,000 rpm, to obtain the precipitation of the virus itself. In preferred embodiments, the first centrifugation at 10,000 rpm, is maintained for 10 minutes and the second at 45,000 rpm, for 20 minutes. These are, of course, only indicative values, it being understood that it remains within the ability of the specialist to modify the centrifugation conditions, to provide for the separation of the cellular constituents and of the viral constituents.

This modification of the purification process results in the production of viral preparations from which the: antigen mentioned can then be isolated more easily, than from virus preparations purified by the previous methods. In any event, the viruses finally obtained by the process of the present application are more easily recognised by serums of patients or of persons who have been exposed to the LAV virus or to morphologically and antigenically similar strains.

The described antigens can themselve be obtained from the above disclosed viruses, by lysis (or other suitable processing) of the latter in the presence of any suitable detergent and by recovery and separation of the antigens released. Advantageously, the lysis of the virus is effected in the presence of aprotinin or of any other agent suitable for inhibiting the action of proteases. The separation of the antigens can then be carried out by any method known in itself ; for example, it is possible to proceed with a separation of the proteins by employing their respectively different migrations in a predetermined gel, the protein sought being then isolated from the zone of the gel in which it would normally be found in an electrophoresis operation under well determined conditions, having regard to its molecular weight. The application discloses antigens separated from the lysate of the abovesaid viruses, due to their affinity for lectins, in particular concanavaline A or lentyl-lectin. The lectin used is preferably immobilised on a solid support, such as the cross linked polymer derived from agarose and marketed under the trade mark SEPHAROSE. After washing of the fixed antigens with a suitable buffer, the antigens can be eluted in any suitable manner, particularly by resorting to a O-methyl-$\alpha$-D-mannopyranoside in solution. -

A more thorough purification of these antigens is described by immunoprecipitation with the serums of patients known to possess antibodies effective against said protein, with concentrated antibody preparations (polyclonal antibodies) or again with monoclonal antibodies, more particularly directed against the antigen according to the invention, in particular that having the molecular weight of 110,000, denoted below by the abbreviation gp110.

Additional characteristics of the invention will appear also in the course of the description which follows of the isolation of a virus according to the invention and of antigens of the virus. Reference will be made to the drawings in which :

Figure 1 is derived from a photographic reproduction of gel strips which have been used to carry out electrophoreses of lysate extracts of T lymphocytes, respectively infected and uninfected (controls) by a LAV suspension.

Figures 2 and 3 are the restriction maps of a complete LAV genome (clone λJ19).

Figures 4a to 4g are the complete sequence of a LAV viral genome.

Figures 5 and 6 show diagrammatically parts of the three possible reading phases of LAV genomic RNA, including the open reading frames (ORF) apparent in each of said reading phases.

I - <u>PRODUCTION OF THE VIRUS AND OF ANTIGENS</u>

T lymphocytes derived from a healthy donor and infected with LAV1, under the conditions described by F. BARRE-SINOUSSI et Coll., on CEM cells derived from a patient afflicted with leukemia and also infected in <u>vitro</u> with LAV1, were kept under cultivation in a medium containing 200 microcuries of $^{35}$S-cystein and devoid of unlabelled cystein. The infected lymphocytes were cultured in a non denaturating medium to prevent the degradation of the antigen sought. The supernatant liquor from the culture medium was then subjected to a first centrifugation at 10,000 rpm for 10 minutes to remove the non viral components, then to a second centrifugation at 45,000 rpm for 20 minutes for sedimenting the virus, the virus pellet was then lysed by detergent in the presence of aprotinin (5 %) particularly under the conditions described in the article of F. BARRE-SINOUSSI et Coll.

The same operation was repeated on lymphocytes taken up from a healthy donor as control.

The various lysates were then immuno-precipitated by serums of patients infected with AIDS or with LAS. Serums originating from healthy donors or of donors infected with other diseases were immunoprecipitated too. The media were then subjected to electrophoreses in a SDS-polyacrylamide gel.

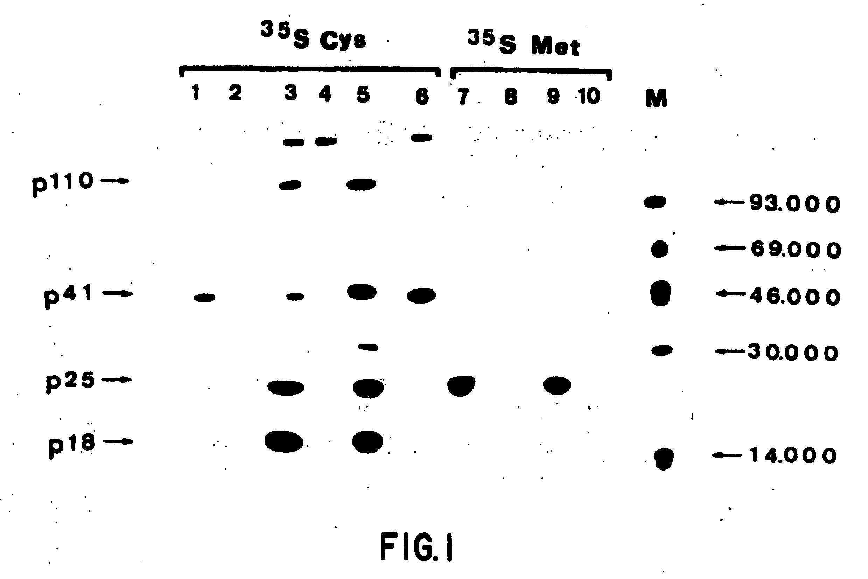

The results are indicated in figure 1. The gel strips numbered from 1 to 6 were obtained from preparations labelled by $^{35}$S-cystein. The strips numbered 7 to 10 show results observed on infected or uninfected lymphocyte preparations labelled with 355-methionine. Finally the strip M corresponds to the migration distances of the standard proteins identified above, whose molecular weights are recalled in the right hand portion of the figure.

The references to the labelled viral proteins appear on the left handside of the figure.

It is noted that columns 7 and 9 show the specific protein p25 of LAV, labelled with $^{35}$s-methionin. The same protein is absent on strips 8 and 10 corresponding to results obtained with a preparation originating from healthy lymphocytes

Columns 3 and 5 correspond to the results which have been observed on preparations obtained from lymphocytes infected and labelled with $^{35}$S-cystein. The proteins p25 and p18, the characteristic core proteins of LAV, and the glycoprotein gp110, also specific of LAV, were also present. Images corresponding to a protein p41 (molecular weight of the order of 41,000) appeared in the various preparations, although less distinctly.

The disclosed virus and antigen can be either precipitated by lectins, particularly concanavaline A, or fixed to a SEPHAROSE-concanavaline A column. Particularly the purification of the envelope glycoproteins can be carried out as follows. This fixation can particularly be carried out by contacting a lysate of the LAV virus dissolved in a suitable buffer with concanavaline-A bound to SEPHAROSE®. A suitable buffer has the following composition :

| | |
|---|---|
| Tris | 10 mM |
| NaCl | 0.15 M |
| $CaCl_2$ | 1 mM |
| $MgCl_2$ | 1 mM |

Detergent marketed under the trade mark TRITON 1 %

PH    7.4

When the fixation has been achieved, the SEPHAROSE®-concanavaline A is washed with a buffer of the same composition, except that the TRITON® concentration is lowered to 0.1 %. The elution is then effected with an 0.2 M O-methyl-α-D-mannopyranoside solution in the washing buffer.

The protein may be further concentrated by immuno-precipitation with antibodies contained in the serums of patients infected with AIDS or with polyclonal antibodies obtained from a serum derived from an animal previously immunised against the "unaltered" virus according to the invention or the abovesaid glycoprotein. The protein can then be recovered by dissociation of the complex by a solution having an adequate content of ionic salt. Preferably the antibody preparation is itself immobilised in a manner known in itself on an insoluble support, for instance of the SEPHAROSE® B type.

It is also possible to resort to monoclonal antibodies secreted by hybridomas previously prepared against gp 110. These monoclonal antibodies, as well as the hybridomas which produce them, also form part of the invention.

A technique for producing and selecting monoclonal antibodies directed against the gp110glycoprotein is described below.

Immunisation of the mice

Groups of Balb/c mice from 6 to 8 weeks old mice were used. One group receives the virus carrying the abovesaid glycoprotein, another a purified glycoprotein gp110. The immunisation procedure, identical for all mice, comprises injecting 10 mg of the antigenic preparation in the presence of Freund complete adjuvant at day 0, then again but in the presence of Freund incomplete adjuvant at day 14 and without adjuvant at days 28 and 42. The three first injections are made intraperitoneally, the fourth intravenously.

**Fusion and culture of the hybrids**

The non secreting myeloma variant 5.53 P3 x 63 Ag8, resistant to azaguanine, itself derived from the MOPC-21 cell-line, is used. Fusion with immunised mouse splenocytes is carried out in the presence of polyethylene-glycol 4000 by the technique of FAZEKAS de st-GROTH and SCHEIDEGGER on the 45th day. The selection of the hybrids in RPMI 16-40 "HAT" medium is carried out in plates having 24 cups (known under the designation COSTAR) by resorting to the same culture techniques.

The hybridomas producing antibodies of adequate specificity are then cloned in plates having 96 cups in the presence of a feeder layer of syngenic thymocytes. The producing clones thus selected are then expanded in 24 cup plates, still in the presence of thymocytes. When the confluence appears in one of the cups, the clone is injected intraperitoneally into a balb/c mouse which had received an injection of PRISTANE 8 days previously and/or kept in liquid culture.

**Demonstration of the anti-LAV antibodies**

Five different techniques enable characterisation of the clones producing antibodies of suitable specificity. In a first stage, the hybrids producing antibodies are determined by an ELISA test revealing mouse immunoglobulins in the supernatant liquors. From this first selection, supernatants are sought which have antibodies directed against viral constituents by means of an ELISA test revealing anti-LAV antibodies, or by immunofluorescence on the virus producing human cells. Finally the supernatant liquors are analysed by radioimmunoprecipitation of virus labelled with cystein and by the Western-Blot technique on viral preparation which permit the determination of the specificities of these anti-LAV antibodies.

RESULTS

Cells obtained from the various fusions are placed under culture in 648 cups. Their microscopic examination shows that the majority of these cups contain a single hybrid clone capable of growing in a "HAT" selective medium. More than 50 % among them produce antibodies giving rise to a positive response under ELISA antivirus examination. The most representative fusions are tested by the Western-Blot technique and several of them are subcloned, taking into account their respective specificities reactivities in antivirus ELISA and their behaviours under the culturing conditions. Those hybrids which are more particularly selected are those which produce antibodies which selectively recognise the viral glycoprotein gp110 having a molecular weight of about 110 KD. All the sub clonings give rise to clones producing antibodies which, after expression, are injected into syngenic mice. Analysis of the specificities of the antibodies present in the different ascites liquids confirms the specificity of the antibodies of said ascites with respect to gp110.

The monoclonal antibodies obtained can themselves be employed to purify proteins containing an antigenic site also contained in gp110. The application also describes these processes of purification as such. This process is advantageously applied to virus lysates or T lymphocyte lysates or other cells producing LAV or the like, when care has been taken to avoid the uncontrolled separation of gp110 during the purification procedure of the virus, prior to lysis thereof. Needless to say that the process can also be applied to any solution containing gp110 or a protein, polypeptide or glycoprotein comprising an antigenic site normally carried by the envelope protein and recognised by the monoclonal antibody. For practising this process, the monoclonal antibodies are advantageously immobilised on a solid support, preferably adapted to affinity chromatography operations. For example, these monoclonal antibodies are fixed to an agarose lattice with three-dimensional cross-linking, marketed under the trade mark SEPHAROSE by the Swedish company PHARMACIA A.G., for example by the cyanogen bromide method.

The invention therefore also relates to a process for separating the antigens concerned, which process comprises contacting a mixture of antigens, including those of interest (for instance a virus lysate or extract), with an affinity column bearing the abovesaid monoclonal antibodies, to selectively fix polypeptides, proteins or glycoproteins selectively recognized by said monoclonal antibodies, recovering the latter by dissociation of the antigen-antibody complex by means of a suitable buffer, particularly a solution of adequate ionic strength, for example of a salt, preferably ammonium acetate (which leaves no residue upon freeze drying of the preparation) or a solution acidified to a pH 2-4 or to a glycine buffer at the same pH and recovering the eluted polypeptides, proteins or glycoproteins.

It is self-evident that the invention relates also to polypeptide fragments having lower molecular weights and carrying antigenic sites recognizable by the same monoclonal antibodies. It is clear to the specialist that the availabililty of monoclonal antibodies recognizing the gp110 glycoprotein gives also access to smaller peptide sequences or fragments containing the common antigenic site or epitope. Fragments of smaller sizes may be obtained by resorting to known techniques. For instance such a method comprises cleaving the original larger polypeptide by enzymes capable of cleaving it at specific sites. By way of examples of such proteins, may be mentioned the enzyme of Staphylococcyus aureus V8, α-chymotrypsine, "mouse sub-maxillary gland protease" marketed by the BOEHRINGER company, Vibrio alginolyticus chemovar iophagus collagenase, which specifically recognises said peptides Gly-Pro and Gly-Ala, etc..

It is also possible to obtain polypeptides or fragments of envelope antigens of the virus, by cloning fragments excised from a cDNA constructed from genomes of LAV variants.

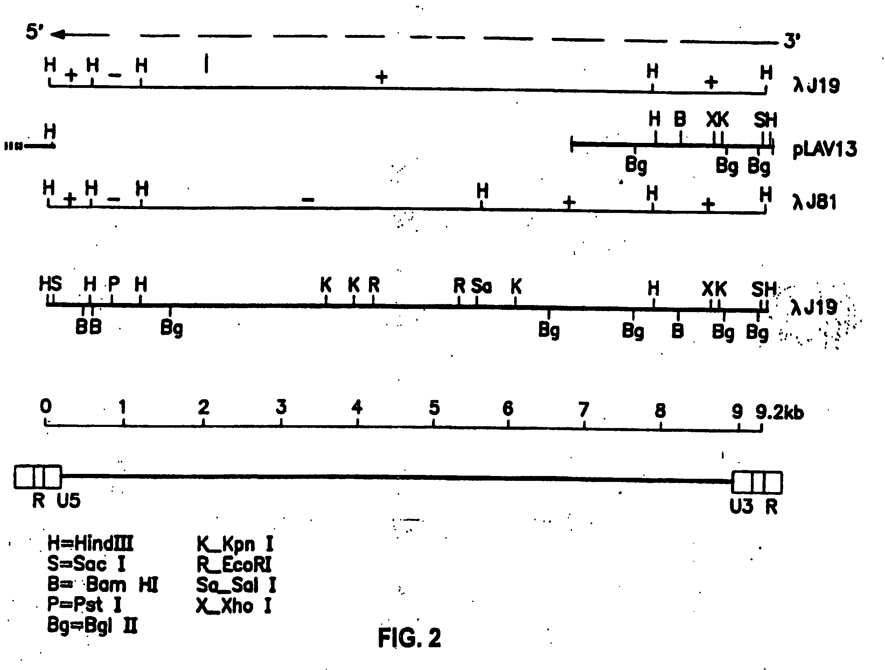

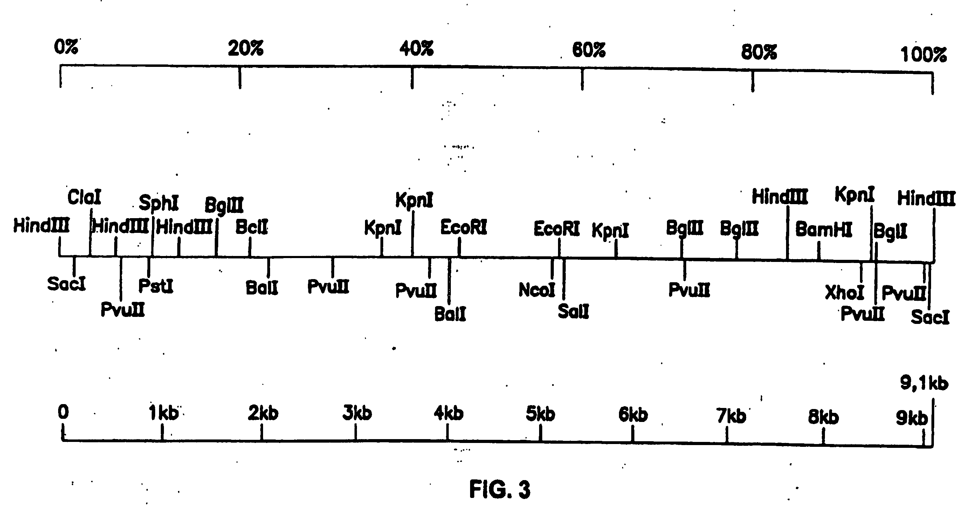

Figures 2 and 3 are restriction maps of such a cDNA comprising a total of 9.1 to 9.2 kb. The polypeptides coded by cDNA fragments located in the region extending between site KpnI (position 6100) and site BgIII (position 9150) of the restriction map of Figure 2. The presence of a characteristic site of an envelope antigen of the LAV virus or the like in any polypeptide expressed (in a suitable host cell transformed beforehand by a corresponding fragment or by a vector containing said fragment) can be detected by any suitable immunochemical means.

Particularly the invention relates more particularly to polypeptides encoded by cDNA fragments defined hereafter. It also relates to the nucleic acid fragments themselves, including a cDNA variant corresponding to a whole LAV retroviral genome, characterized by a series of restriction sites in the order hereafter (from the 5' end to the 3' end).

The coordinates of the successive sites of the whole LAV genome (see also restriction map of λJ19 in fig. 2) are indicated hereafter too, with respect to the Hind III site (selected as of coordinate 1) which is located in the R region. The coordinates are estimated with an accuracy of $\pm$ 200 bp :

| | |
|---|---|
| Hind III | 0 |
| Sac I | 50 |
| Hind III | 520 |

(continued)

| | |
|---|---|
| Pst I | 800 |
| Hind III | 1 100 |
| Bgl II | 1 500 |
| Kpn I | 3 500 |
| Kpn I | 3 900 |
| Eco RI | 4 100 |
| Eco RI | 5 300 |
| Sal I | 5 500 |
| Kpn I | 6 100 |
| Bgl II | 6500 |
| Bgl II | 7 600 |
| Hind III | 7 850 |
| Bam HI | 8 150 |
| Xho I | 8 600 |
| Kpn I | 8 700 |
| Bgl II | 8 750 |
| Bgl II | 9 150 |
| Sac I | 9 200 |
| Hind III | 9 250 |

Another DNA variant disclosed herein optionally contains an additional Hind III approximately at the 5 550 coordinate.

Reference is further made to fig. 2 which shows a more detailed restriction map of said whole-DNA (λJ19)

An even more detailed nucleotidic sequence of a preferred DNA according to the invention is shown in figs. 4a-4e hereafter.

The invention further relates to other preferred DNA fragments and polypeptide sequences (glycosylated or not glycosylated) which will be referred to hereafter.

<u>SEQUENCING OF LAV</u>

The sequencing and determination of sites of particular interest were carried out on a phage recombinant corresponding to λJ19 disclosed in the abovesaid British Patent application Nr. 84 23659. A method for preparing it is disclosed in that application.

The whole recombinant phage DNA of clone λJ19 (disclosed in the earlier application) was sonicated according to the protocol of DEININGER (1983), Analytical Biochem. 129, 216. The DNA was repaired by a Klenow reaction for 12 hours at 16°C. The DNA was electrophoresed through 0.8 % agarose gel and DNA in the size range of 300-600 bp was cut out and electroeluted and precipitated. Resuspended DNA (in 10 mM Tris, pH 8 ; 0,1 mM EDTA) was ligated into M13mp8 RF DNA (cut by the restriction enzyme SmaI and subsequently alkaline phosphated), using T4 DNA- and RNA-ligases (Maniatis T et al (1982) - Molecular cloning - Cold Spring Harbor Laboratory). An <u>E</u>. coli strain designated as TG1 was used for further study. This strain has the following genotype : Δlac pro, supE, thi. F'traD36, proAB, lacIq, ZΔM15,r⁻

This <u>E. coli</u> TGI strain has the peculiarity of enabling recombinants to be recognized easily. The blue colour of the cells transfected with plasmids which did not recombine with a fragment of LAV DNA is not modified. To the contrary cells transfected by a recombinant plasmid containing a LAV DNA fragment yield white colonies. The technique which was used is disclosed in Gene (1983), 26, 101

This strain was transformed with the ligation mix using the Hanahan method (Hanahan D (1983) J.Mol. Biol. 166, 557). Cells were plated out on tryptone-agarose plate with IPTG and X-gal in soft agarose. White plaques were either picked and screened or screened directly <u>in situ</u> using nitrocellulose filters. Their DNAs were hybridized with nick-translated DNA inserts of pUC18 Hind III subclones of λJ19. This permitted the isolation of the plasmids or subclones of λ which are identified in the table hereafter. In relation to this table it should also be noted that the designation of each plasmids is followed by the deposition number of a cell culture of <u>E</u>. coli TGI containing the corresponding plasmid at the "Collection Nationale des Cultures de Micro-organismes⁻ (C.N.C.M.) of the Pasteur Institute in Paris, France. A non-transformed TGI cell line was also deposited at the C.N.C.M. under Nr. I-364. All these deposits took place on November 15, 1984. The sizes of the corresponding inserts derived from the LAV genome have also been indicated.

## TABLE

### Essential features of the recombinant plasmids

| - pJ19 - 1 plasmid | (I-365) | 0.5 kb |
|---|---|---|
| Hind III - Sac I - Hind III - | | |
| - pJ19- 17 plasmid | (I-367) | 0.6 kb |
| Hind III - Pst 1 - Hind III | | |
| - pJ19 - 6 plasmid | (I-366) | 1.5 kb |
| Hind III (5') | | |
| Ban HI | | |
| Xho I | | |
| Kpn I | | |
| Bgl II | | |
| Sac I (3') | | |
| Hind III | | |
| - pJ19-13 plasmid | (I-368) | 6.7 kb |
| Hind III (5') | | |
| Bgl II | | |
| Kpn I | | |
| Kpn I | | |
| Eco RI | | |
| Eco RI | | |
| Sal I | | |
| Kpn I | | |
| Bgl II | | |
| Bgl II | | |
| Hind III(3') | | |

Positively hybridizing M13 phage plates were grown up for 5 hours and the single-stranded DNAs were extracted.

M13mp8 subclones of λJ19 DNAs were sequenced according to the dideoxy method and technology devised by Sanger et al (Sanger et al (1977), Proc. Natl. Acad. Sci. USA, 74, 5463 and M13 cloning and sequencing handbook, AMERSHAM (1983). the 17-mer oligonucleotide primer $\alpha$-$^{35}$SdATP (400Ci/mmol, AMERSHAM), and 0.5X-5X buffer gradient gels (Biggen M.D. et al (1983, Proc. Natl. Acad. Sci. USA, 50, 3963) were used. Gels were read and put into the computer under the programs of Staden (Staden R. (1982), Nucl. Acids Res. 10, 4731). All the appropriate references and methods can be found in the AMERSHAM M13 cloning and sequencing handbook.

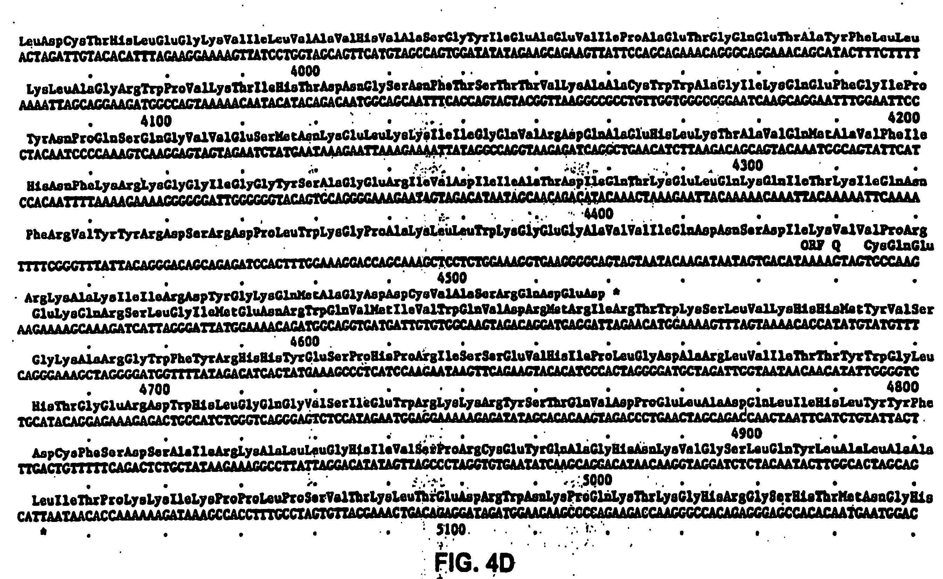

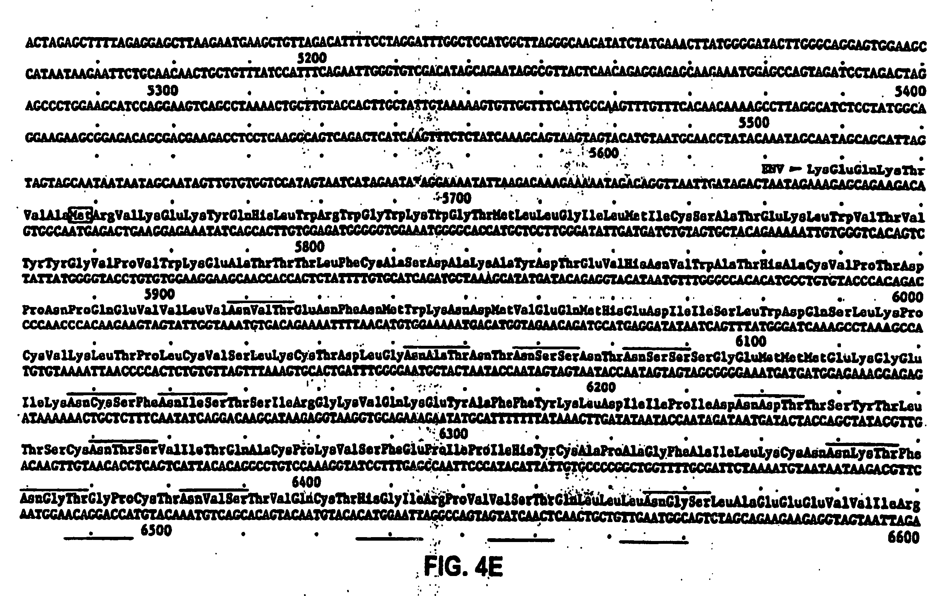

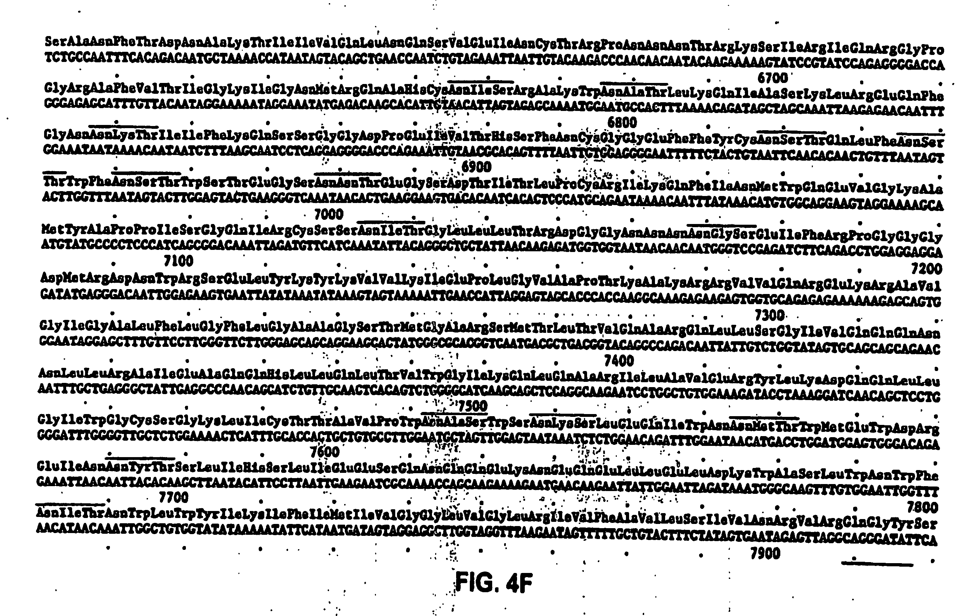

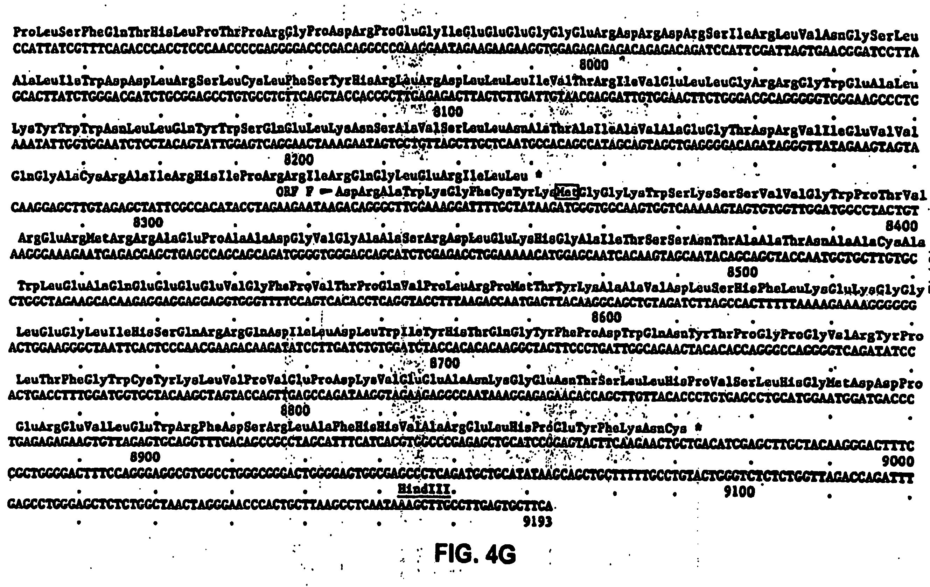

The complete DNA sequence of λJ19 (also designated as LAV-Ia) is shown in figs. 4 to 4e.

The sequence was reconstructed from the sequence of phage λJ19 insert. The numbering starts at the cap site which was located experimentally (see hereafter). Important genetic elements, major open reading frames and their predicted products are indicated together with the HindIII cloning sites. The potential glycosylation sites in the env gene are overlined. The NH$_2$-terminal sequence of p25$^{gag}$ determined by protein microsequencing is boxed.

Each nucleotide was sequenced on average 5.3 times : 85 % of the sequence was determined on both strands and the remainder sequenced at least twice from independent clones. The base composition is T, 22.2 % ; C, 17.8 % ; A, 35.8 % ; G, 244.2 % ; G + C, 42 %. The dinucleotide GC is greatly under represented (0,9%) as common amongst eukaryotic sequences (Bird 1980).

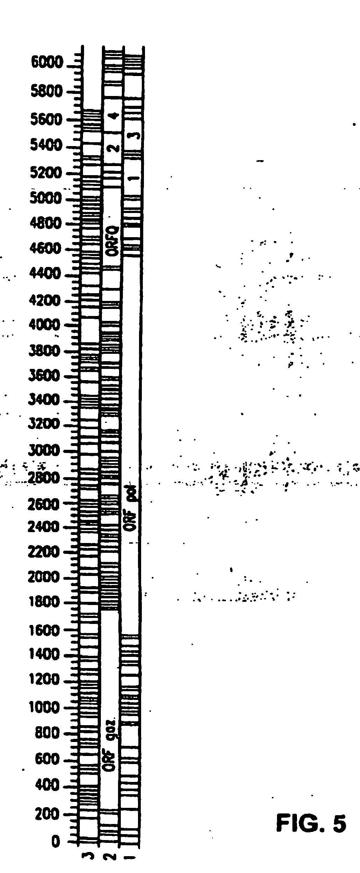

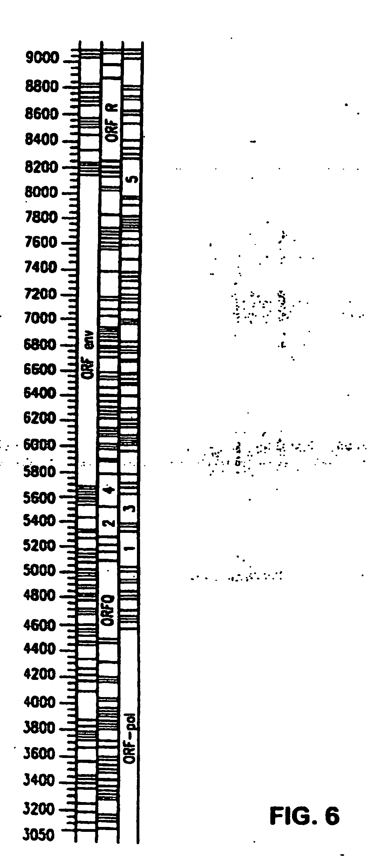

Figs. 5 and 6 provide a diagrammatized representation of the lengths of the successive open reading frames corresponding to the successive reading phases (also referred to by numbers "1", "2" and "3" appearing in the left handside part of fig. 5). The relative positions of these open reading frames (ORF) with respect to the nucleotidic structure of the LAV genome is referred to by the scale of numbers representative of the respective positions of the corresponding nucleotides in the DNA sequence. The vertical bars correspond to the positions of the corresponding stop codons.

The following genes and DNA fragments can be distinguished on the different reading frames shown. Reference is then also made to the proteins or glycoproteins encoded by said genes and fragments, particularly to ORFs sequences such as ORF-O and ORF-F.

The application discloses DNA sequences which provide open reading frames defined as ORF-Q, ORF-F and as "1", "2", "3", "4", "5", the relative positions of which appear more particularly in figs. 2 and 3.

These ORFs have the following locations :

| | phase | | start | | stop | |
|---|---|---|---|---|---|---|
| ORT-Q | phase | 1 | start | 4554 | stop | 5162 |
| ORF-F | - | 2 | - | 8324 | - | 8972 |
| ORF-1 | - | 1 | - | 5105 | - | 5392 |
| ORF-2 | - | 2 | - | 5349 | - | 5591 |
| ORF-3 | - | 1 | - | 5459 | - | 5692 |
| ORF-4 | - | 2 | - | 5595 | - | 5849 |
| ORF-5 | - | 1 | - | 8042 | - | 8355 |

## ORFs O and F

The viral (+) strand of the LAV genome was found to contain the statutory retroviral genes encoding the core structural proteins (gag), reverse transcriptase (pol) and envelope protein (env), and two extra open reading frames (orf) which we call Q and F (Table 1). The genetic organization of LAV. 5' LTR-gag-pol-O-env-F-3'LTR. is unique. Whereas in all replication competent retroviruses pol and env genes overlap, in LAV they are separated by orf 0 (192 amino acids) followed by four small (<100 triplets) orf. The orf F (206 amino acids) slightly overlaps the 3' end of env and is remarkable in that it is half encoded by the U3 region of the LTR.

Such a structure places LAV clearly apart from previously sequenced retroviruses (Fig. 2). The (-) strand is apparently non coding. The additional HindIII site of the LAV clone λJ81 (with respect to λJ19) maps to the apparently non-coding region between Q and env (positions 5166-5745). Starting at position 5501 is a sequence (AAGCCT) which differs by a single base (underlined) from the HindIII recognition sequence. It is to be anticipated that many of the restriction site polymorphism between different isolates will map to this region. Clone λJ81 has also been referred to in British application Nr. 84 23659 filed on September 15, 1984.

### O and F :

The nucleotide positions of their respective extremities are given in Table 1 hereafter.

The location of orf Q is without precedent in the structure of retroviruses. Orf F is unique in that it is half encoded by the U3 element of the LTR. Both orfs have "strong" initiator codons (Kozak 1984) near their 5" ends and can encode proteins of 192 aminoacids (MW calc = 22487) and 206 aminoacids (MWcalc = 23316) respectively. Both putative proteins are hydrophilic (pO 49 % polar. 15.1 % Arc + Lys : pF 46 % polar, 11 % Ara + Lys) and are therefore unlikely to be associated directly with membrane. The function for the putative proteins pQ and pF cannot be predicted as no homology was found by screening protein sequence data banks. Between orf F and the pX protein of HTLV-1 there is no detectable homology. Furthermore their hydrophobicity/hydrophilicity profiles are completely different. It is known that retroviruses can transduce cellular genes notably proto-ancogenes (Weinberg 1982)). We suggest that orfs Q and F represent exogenous genetic material and not some vestige of cellular DNA because (I) LAV DNA does not hybridize to the human genome under stringent conditions (Alizon et al., 1984), (II) their codon usage is comparable to that of the gag, pol and env genes (data not shown).

The organization of a reconstructed LTR and viral flanking elements are shown schematically in FiG. 6. The LTR is 63B be lone and displays usual features5Chen and Barker 1984) : (I) It is bounded by an inverted repeat (5'ACTG) including the conserved TG dinucleotide (Temin 1981). (II) Adjacent to 5' LTR is the tRNA primer binding site (PBS), complementary to $tRNA^{lys}_{3}$ (Raba et al., 1979), (III) adjacent to 3'LTR is a perfect 15 bp polypurine tract. The other three polypurine tracts observed between nucleotides 8200-8800 are not followed by a sequence which is complementary to that just proceeding the PBS. The limits of U5. R and U3 elements were determined as follows. U5 is located between PBS and the polyadenylation site established from the sequence of the 3' end of oligo(dT)-primed LAVcDNA (Alizon et al., 1984). Thus U5 is 84 bos long. The length of R+U5 was determined by synthesizing tRNA-primed LAV cDNA. After alkaline hydrolysis of the primer, R+U5 was found to be 181 $\pm$ 1 bp. Thus R is 97 bps long and the capping site at its 5' end can be located. Finally U3 is 456 bp long. The LAV LTR also contains characteristic regulatory elements : a polyadenylation signal sequence AATAAA 19 bp from the R-U5 junction and the sequence ATATAAG which is very likely the TATA box, 22 bps 5' of the cap site. There are no longer direct repeats within the LTR. Interestingly the LAV LTR shows some similarities to that of the mouse mammary tumour virus (MMTV) Donehower et al., 1981). They both use $tRNA^{lys}_{3}$ as a primer for (-) strand synthesis whereas all other exogenous mammalian retroviruses known to date use tRNA$^{pro}$ (Chen and Barker 1984). They possess very similar polypurine tracts (that of LAV is AAAAGAAAAGGGGGG while that of MMTV is AAAAAAGAAAAAGGGGG). It is probable that the viral (+) strand synthesis is discontinuous since the polypurine tract flanking the U3 element of the 3' LTR is found exactly duplicated in the 3' end of orf pol, at 4331-4336. In addition, MMTV and LAV are exceptionnal in that the U3 element can encode an orf. In the case of MMTV. U3 contains the whole orf while in LAV, U3 contains 110 codons of the 3' half of orf F.

The LAV long terminal repeat (LTR) is diagrammatically represented in Fig. 6. As mentioned the LTR was reconstructed from the sequence of λJ19 by juxtaposing the sequences adjacent to the HindIII cloning sites.

Sequencing of oligo(dT) primed LAV DNA clone pLAV75 (Alizon et al.. 1984) rules out the possibility of clustered HindIII sites in the R region of LAV. LTR are limited by an invertd repeat sequence (IR). Both of the viral elements flanking the LTR have been represented = tRNA primer binding site (PBS) for 5' LTR and polypurine track (PU) for 3' LTR. Also indicated are a putative TATA box, the cap site, polydenylation signal (AATAAA) and polyadenylation site (CAA). The location of the open reading frame F (648 nucleotides) is shown above the LTR schema.

The LTR (long terminal repeats) can also be defined as lying between position 8560 and position 160 (end extending over position 9097/1). As a matter of fact the end of the genome is at 9097 and, because of the LTR structure of the retrovirus, links up with the beginning of the sequence :

**Hind III**

**CTCAATAAAGCTTGCCTTG**

9097  1

Table 1 sums up the locations and sizes of viral open reading frames. The nucleotide coordinates refer to the first base of the first triplet (1st triplet), of the first methionine initiation codon (Met) and of the stop codon (stop). The number of aminoacids and calculated molecular weights are those calculated for unmodified precursor products starting at the first methionine through to the end with the exception of pol where the size and MW refer to that of the whole orf.

Table I : Location and sizes of viral open reading frames.

| orf | Ist triplet | Met | stop | No amino acids | MWcalc |
|-----|-------------|-----|------|----------------|--------|
| gag | 312 | 336 | 1836 | 500 | 55841 |
| pol | 1631 | 1934 | 4640 | (1003) | (113629) |
| art Q | 4354 | 4387 | 5163 | 192 | 22487 |
| env | 5746 | 5767 | 8350 | 861 | 97376 |
| orl F | 8324 | 8354 | 8972 | 206 | 23316 |

The application discloses more particularly all the DNA fragments which have been more specifically referred to hereabove and which correspond to open reading frames. It will be understood that the man skilled in the art will be able to obtain them all, for instance by cleaving an entire DNA correspondinq to the complete genome of a LAV species, such as by cleavage by a partial or complete digestion thereof with a suitable restriction enzyme and by the subsequent recovery of the relevant fragments. The different DNAs disclosed above can be resorted to also as a source of suitable fragments. The techniques disclosed hereafter for the isolation of the fragments which were then included in they plasmids referred to hereabove and which were then used for the DNA sequencing can be used.

Of course other methods can be used. Some of them have been examplified in British Application Nr. 8423659 filed on September 19, 1984. Reference is for instance made to the following methods.

a) DNA can be transfected into mammalian cells with appropriate selection markers by a variety of techniques, calcium phosphate precipitation, polyethylene glycol, protoplast-fusion, etc..
b) DNA fragments corresponding to genes can be cloned into expression vectors for E. coli, yeast- or mammalian cells and the resultant proteins purified.
c) The provival DNA can be "shot-gunned" (fragmented) into procaryotic expression vectors to generate fusion polypeptides. Recombinant producing antigenically competent fusion proteins can be identified by simply screening the recombinants with antibodies against LAV antigens.

The application further describes more specifically DNA recombinants, particularly modified vectors, including any of the preceding DNA sequences and adapted to transform corresponding microorganisms or cells, particularly eucaryotic cells such as yeasts, for instance Saccharomyces cerevisiae, or higher eucaryotic cells, particularly cells of mammals, and to permit expression of said DNA sequences in the corresponding microorganisms or cells.

More particularly the application discloses such modified DNA recombinant vectors modified by the abovesaid DNA sequences and which are capable of transforming higher eucaryotic cells particularly mammalian cells. Preferably any of the abovesaid sequences are placed under the direct control of a promoter contained in said vectors and which is recognized by the polymerases of said cells, such that the first nucleotide codons expressed correspond to the first triplets of the above-defined DNA-sequences. Accordingly this application also describes the corresponding DNA fragments which can be obtained from LAV genomes or corresponding cDNAs by any appropriate method. For instance such a method comprises cleaving said LAV genomes or cDNAs by restriction enzymes preferably at the level of restriction sites surrounding said fragments and close to the opposite extremities respectively thereof, recovering and identifying the fragments sought according to sizes, if need be checking their restriction maps or nucleotide sequences (or by reaction with monoclonal antibodies specifically directed against epitopes carried by the polypeptides encoded by said DNA fragments), and further if need be, trimming the extremities of the fragment, for instance by an exonucleolytic enzyme such as Bal31, for the purpose of controlling the desired nucleotide-sequences of the extremities of said DNA fragments or, conversely, repairing said extremities with Klenow enzyme and possibly ligating the latter to synthetic polynucleotide fragments designed to permit the reconstitution of the nucleotide extremities of said fragments. Those fragments may then be inserted in any of said vectors for causing the expression of the corresponding polypeptide by the cell transformed therewith. The corresponding polypeptide can then be recovered from the transformed cells, if need be after lysis thereof, and purified, by methods such as electrophoresis. Needless to say that all conventionnal methods for per-forming these operations can be resorted to.

The application also discloses more specifically cloned probes which can be made starting from any DNA fragment described herein thus to recombinant DNAs containing such fragments, particularly any plasmids amplifiable in procaryotic or eucaryotic cells and carrying said fragments.

Using the cloned DNA fragments as a molecular hybridization probe - either bv labelling with radionucleotides or with fluorescent reagents - LAV virion RMA may be detected directly in the blood, body fluids and blood products (e.g. of the antihemophylic factors such as Factor VIII concentrates) and vaccines, i.e. hepatitis B vaccine. It has already been shown that whole virus can be detected in culture supernatants of LAV producing cells. A suitable method for achieving that detection comprises immobilizing virus onto a support, e.g. nitrocellulose filters, etc., disrupting the virion and hybridizing with labelled (radiolabelled or "cold" fluorescent- or enzyme-labelled) probes. Such an approach has already been developed for Hepatitis B virus in peripheral blood (according to SCOTTO J. et al. Hepatology (1983), 3, 379-384).

Probes disclosed in the application can also be used for rapid screening of genomic DNA derived from the tissue of patients with LAV related symptoms, to see if the proviral DNA or RNA is present in host tissue and other tissues.

A method which can be used for such screening comprises the following steps : extraction of DNA from tissue, restriction enzyme cleavage of said DNA, electrophoresis of the fragments and Southern blotting of genomic DNA from tissues, subsequent hybridization with labelled cloned LAV proviral DNA. Hybridization in situ can also be used.

Lymphatic fluids and tissues and other non-lymphatic tissues of humans, primates and other mammalian species can also be screened to see if other evolutionnary related retrovirus exist. The methods referred to hereabove can be used although hybridization and washings would be done under non stringent conditions.

The DNAs or DNA fragments disclosed in the application can be used for achieving expression LAV viral antigens for diagnostic purposes.

The invention relates generally to the polypeptides themselves, whether svnthetized chemically isolated from viral preparation or, expressed by the different DNAs of the invention particularly by the ORFs or fragments thereof, in appropriate hosts, particularly procaryotic or eucaryotic hosts, after transformation thereof with a suitable vector previously modified by the corresponding DNAs.

More generally, the invention also relates to any of the polypeptide fragments (or molecules, particularly glycoproteins having the same polypeptidic backbone as the polypeptides mentioned hereabove) bearing an epitope characteristic of a LAV protein or glycoprotein, which polypeptide or molecule then has N-terminal and C-terminal extremities respectively either free or, independently from each other, covalently bound to aminoacids other than those which are normally associated with them in the larger polypeptides or glycoproteins of the LAV virus, which last mentioned aminoacids are then free or belong to another polypeptidic sequence. Particularly the invention relates to hybrid polypeptides containing any of the epitope-bearing-polypeptides which have been defined more specifically hereabove, recombined with other polypeptides fragments normally foreign to the LAV proteins, having sizes sufficient to provide for an increased immunogenicity of the epitope-bearing-polypeptide yet, said foreign polypeptide fragments either being immunogenically inert or not interfering with the immunogenic properties of the epitope-bearing-polypeptide.

Such hybrid polypeptides which may contain up to 150, even 250 aminoacids usually consist of the expression products of a vector which contained ab initio a nucleic acid sequence expressible under the control of a suitable promoter or replicon in a suitable host, which nucleic acid sequence had however beforehand been modified by insertion therein of a DNA sequence encoding said epitope-bearing-polypeptide.

Said epitope-bearing-polypeptides, particularly those whose N-terminal and C-terminal aminoacids are free, are also accessible by chemical synthesis, according to technics well known in the chemistry of proteins.

The synthesis of peptides in homogeneous solution and in solid phase is well known.

In this respect, recourse may be had to the method of synthesis in homogeneous solution described by Houbenweyl in the work entitled "Methoden der Organischen Chemie" (Methods of Organic Chemistry) edited by E. WUNSCH.. vol. 15-I and II. THIEME. Stuttgart 1974.

This method of synthesis consists of successively condensing either the successive aminoacids in twos, in the appropriate order or successive peptide fragments previously available or formed and containing already several aminoacyl residues in the appropriate order respectively. Except for the carboxyl and aminogroups which will be engaged in the formation of the peptide bonds, care must be taken to Protect beforehand all other reactive groups borne by these aminoacyl groups or fragments. However, prior to the formation of the peptide bonds, the carboxyl groups are advantageously activated, according to methods well known in the synthesis of peptides. Alternatively, recourse may be had to coupling reactions bringing into play conventional coupling reagents, for instance of the carbodiimide type, such as 1-ethyl-3-(3-dimethyl-aminopropyl)-carbodiimide. When the aminoacid group used carries an additional amine group (e.g. lysine) or another acid function (e.g. glutamic acid), these groups may be protected by carbobenzoxy or t-butyloxtrcarbonyl groups, as regards the amine groups, or by t-butylester groups, as regards the carboxylic groups. Similar procedures are available for the protection of other reactive groups. For example. SH group (e.g. in cysteine) can be protected by an acetamidomethyl or paramethoxvbenzvl group.

In the case of progressive synthesis, aminoacid by aminoacid, the synthesis starts preferably by the condensation of the C-terminal aminoacid with the aminoacid which corresponds to the neighboring aminoacyl group in the desired sequence and so on, step by step, up to the N-terminal aminoacid. Another preferred technique can be relied upon is that described by R.D. Merrifield in "solid phase peptide synthesis" (J. Am. Chem. Soc., 45, 2149-2154).

In accordance with the Merrifield process, the first C-terminal aminoacid of the chain is fixed to a suitable porous polymeric resin, by means of its carboxylic group, the amino group of said aminoacid then being protected, for example by a t-butyloxycarbonyl group.

When the first C-terminal aminoacid is thus fixed to the resin, the protective group of the amine group is removed by washing the resin with an acid, i.e. trifluoroacetic acid, when the protective group of the amine group is a t-butyloxycarbonyl group.

Then the carboxylic group of the second aminoacid which is to provide the second aminoacyl group of the desired peptidic sequence, is coupled to the deprotected amine group of the C-terminal aminoacid fixed to the resin. Preferably, the carboxyl group of this second aminoacid has been activated, for example by dicyclohexylcarbodiimide, while its amine Group has been protected, for example by a t-butyloxycarbanyl group. The first part of the desired peptide chain, which comprising the first two aminoacids, is thus obtained. As previously, the amine Group is then deprotected, and one can further proceed with the fixing of the next aminoacyl group and to forth until the whole peptide sought is obtained.

The protective groups of the different side Groups, if any, of the peptide chain so formed can then be removed. The peptide sought can then be detached from the resin, for example, by means of hydrofluoric acid, and finally recovered in pure form from the acid solution according to conventional procedures.

As regards the peptide sequences of smallest size and bearing an epitope or imunogenic determinant, and more particularly those which are readily accessible by chemical synthesis, it may be required, in order to increase their in vivo immunogenic character, to couple or "conjugate" them covalentlv to a physiologically acceptable and non toxic carrier molecule.

By way of examples of carrier molecules or macromolecular supports which can be used for making the conjugates according to the invention, will be mentioned natural proteins, such as tetanic toxoid, ovalbumin, serum-albumins, hemocyanins, etc.. Synthetic macromolecular carriers, for example polysines or poly(D-L-alanine)-poly(L-Lysine)s, can be used too

Other types of macromolecular carriers which can be used, which generally have molecular weights higher than 20,000, are known from the literature.

The conjugates can be synthesized by known processes, such as described by Frantz and Robertson in "Infect. and Immunity", 33, 193-198 (1981), or by P.E. Kauffman in Applied and Environmental Microbiology. October 1981. Vol. 42, n° 4, 611-614

For instance the following coupling agents can be used : glutaric aldehyde, ethyl chloroformate, water-soluble carbodiimides (N-ethyl-N'(3-dimethylaminopropyl) carbodiimide. HCl), diisocyanates, bis-diazobenzidine, di- and trichloro-s-triazines, cyanogen bromides, benzaquinone, as well as coupling agents mentioned in "Scand. J. Immunol., 1978, vol. 8. p. 7-23 (Avrameas, Ternynck. Guesdon).

Any coupling process can be used for bonding one or several reactive, groups of the peptide, on the one hand, and one or several reactive groups of the carrier on the other hand. Again coupling is advantageously achieved between carboxyl and amine groups carried by the peptide and the carrier or vice-versa in the presence of a coupling agent of the type used in protein synthesis, i.e. 1-ethyl-3-(3-dimethylaminopropyl)-carbodiimide, N-hydroxybenzotriazole, etc.. Coupling between amine groups respectively borne by the peptide and the carrier can also be made with glutaraldehyde, for instance, according to the method described by BOQUET. P. et al. (1982) Molec. Immunol., 12. 1441-1549, when the carrier is hemocyanin.

The immunogenicity of epitope-bearing-peptides can also be reinforced, by oligomerisation thereof, for example in the presence of glutaraldehyde or any other suitable coupling agent. In particular, the invention relates to the water soluble immunogenic oligomers thus obtained, comprising particularly from 2 to 10 monomer units.

The glycoproteins, proteins and polypeptides (generally designated hereafter as "antigens" of this invention, obtained in a purified state by DNA recombinant technology are useful in processes for the detection of the presence of anti-LAV antibodies in biological media, particularly biological fluids such as sera from man or animal, particularly with a view of possibly diagnosing LAS or AIDS.

Particularly the invention discloses an in vitro process of diagnosis making use of an envelope glycoprotein (or of a polypeptide bearing an epitope of this glycoprotein) for the detection of anti-LAV antibodies in the serums of persons who carry them. Other polypeptides - particular those carrying an epitope of a core protein - can be used too, according to the invention.

A preferred embodiment of the process of the invention comprises

- depositing a predetermined amount of one or several of' said antigens in the cups of a titration miroplate :

- introducing of increasing dilutions of the biological fluid, i.e. serum to be diagnosed into these cups :

- incubating the microplate :

- washing carefully the microplate with an appropriate buffer :

- adding into the cups specific labelled antibodies directed against blood immunoglobulins and

- detecting the antigen-antibody-complex formed, which is then indicative of the presence of LAV antibodies in the biological fluid.

Advantageously the labelling of the anti-immunoqlobulin antibodies is achieved by an enzyme selected from among those which are capable of hydrolysing a substrate, which substrate undergoes a modification of its radiation-absorption, at least within a predetermined band of wavelenghts. The detection of the substrate, pre-ferably comparatively with respect to a control, then provides a measurement of the potential risks or of the effective presence of the disease.

Thus preferred methods immuno-enzymatic or also immunofluorescent detections, in particular according to the ELISA technique. Titrations may be determinations by immunofluorescence or direct or indirect immuno-enzymatic determinations. Quantitative titrations of antibodies on the serums studied can be made.

The invention also relates to the diagnostic kits themselves for the in vitro detection of antibodies against the LAV virus, which kits comprise anv of the polypeptides identified herein, and all the biological and chemical reagents, as well as equipment, necessary for peforming diagnostic assays. Preferred kits comprise all reagents required for carrying out ELISA assays. Thus preferred kits will include, in addition to any of said polypeptides, suitable buffers and anti-human = immunoglobulins, which anti-human immunoglobulins are labelled either by an immunofluorescent molecule or bv an enzyme. In the last instance preferred kits then also comprise a substrate hvdrolvsable by the enzyme and providing a signal, particularly modified absorption of a radiation, at least in a determined wavelength, which signal is then indicative of the presence of antibody in the biological fluid to be assayed with said kit.

The application also describes vaccine com-positions whose active principle is to be constituted by any of the antigens, i.e. the hereabove disclosed polypeptides whole antigens. fusion polypeptides or oligopeptides in association with a suitable pharmaceutical or physiologically acceptable carrier.

A first type of preferred active principle is the gp110 immunogen.

Other preferred active principles to be considered in that fields consist of the peptides containing less than 250 aminoacid units, preferably less than 150. as deducible for the complete genomas of LAV, and even more preferably those peptides which contain one or more groups selected from Asn-X-Ser and Asn-X-Ser as defined above

By way of example having no limitative character, there may be mentioned that suitable dosaoes of the vaccine compositions are those which are effective to elicit antibodies in vivo, in the host, Particularly a human host. Suitable doses range from 10 to 500 micrograms of polypeptide, protein or glycoprotein per kg, for instance 50 to 100 micrograms per kg.

The different peptides according to this invention can also be used themselves for the production of antibodies, preferably monoclonal antibodies specific of the different peptides respectively. For the production of hybridomas secreting said monoclonal antibodies, conventional production and screening methods are used. These monoclonal antibodies, which themselves are part of the invention then provide very useful tools for the identification and even determination of relative proportions of the different polypeptides or proteins in biological samples, particularly human samples containing LAV or related viruses.

The application describes the hosts (procaryotic or eucaryotic cells) which are transformed by the above mentioned recombinants and which are capable of expressing said DNA fragments.

Finally the application also describes vectors for the transformation of eucaryotic cells of human origin, particularly lymphocytes, the polymerases of which are capable of recognizing the LTRs of LAV. Particularly said vectors are characterized by the presence of a LAV LTR therein, said LTR being then active as a promoter enabling the efficient transcription and translation in a suitable host of a DNA insert coding for a determined protein placed under its controls.

Needless to say that the application extends to all variants of genomes and corresponding DNA fragments (ORFs) having substantially equivalent properties, all of said genomes belonging to retroviruses which can be considered as equivalents of LAV.

REFERENCES

Alizon, M., Sonigo, P., Barre-Sinoussi, F., Chermann, J.C., Tiollais, P., Montagnier, L. and Wain-Hobson, S. (1984). Molecular cloning of lymphadenopathy-associated virus. Nature, in press.

Arya, S.K., Gallo, R.C., Hahn, B.H., Shaw, G.M., Popovic, M., Salahuddin, S.Z. and Wong-Staal, F. (1984). Homology of genome of AIDS-associated virus with genomes of human T-cell leukemia lymphoma viruses. Science 225, 927-930.

Barré-Sinoussi, F., Chermann, J.C., Rey, F., Nugeybe, M.T., Chamaret, S., Gruest, J., Dauguet, C., Axler-Blin, C., Vézinet-Brun F., Rouzioux, C., Rozenbaum, W. and Montagnier, L. (1983). Isolation of a T-lymphotropic retrovirus from a patient at risk of Acquired Immune Deficiency Syndrome (AIDS). Science 220, 868-870.

Biggen, M.D., Gibson, T.J. and Hong, G.F. (1983). Buffer gradient gels and 35S label as an aid to rapid DNA sequence determination. Proc. Natl. Acad. Sci. USA 80, 3963-3965.

Bird, A.P. (1980). DNA methylation and the frequency of CpG in animal DNA. Nucl. Acids Res. 8, 1499-1504.

Brun-Vézinet, F., Rouzioux, C., Barré-Sinoussi, F., Klatzmann, D., Saimot, A.G., Rozembaum, W., Montagnier, L. and Chermann, J.C. (1984). Detection of IgG antibodies to lymphadenopathy associated virus (LAV) by ELISA, in patients with acquired immuno-deficiency syndrome of lymphadenopathy syndrome. Lancet I, 1253-1256.

Chen, H.R. and Barker, W.C. (1984). Nucleotide sequences of the retroviral long terminal repeats and their adjacent regions. Nucl. Acids Res. 12, 1767-1773

Chen, I.S.Y., Mc Laughlin, J., Gasson, J.C., Clark, S.C. and Golde, D.W. (1983). Molecular characterization of genome of a novel human T-cell leukaemia virus. Nature 303, 502-505

Chiu, I-M., Callahan, R., Tronick, S.R., Scholm, J. and Aaronson, S.A. (1984). Major pol gene progenitors in the evolution of oncornaviruses Science 223, 364-370:

Cianciolo, G.J., Kipnis, R.J. and Snyderman, R. (1984). Similarity between p15E of murine and feline viruses and p21 of HTLV. Nature 311, 515.

Daly, H.M. and Scott, G.L. (1983). Fatal AIDS in a haemophiliae in the U.K. Lancet II, 1190.

Dittmar, K.J. and Moelling, K. (1978). Biochemical properties of p15-associated protease in an avion RNA tumor virus. J. Virol. 28, 106-113.

Donehower, L.A., Huang, A.L. and Hager, G.L. (1981). Regulatory and coding potential of the mouse mammary tumour virus long terminal redundancy. J. Virol. 37, 226-238.

Gottlieb, M.S., Schroff, R., Schanler, H.M., Weisman, J.D., Fan P.T., Wolf R.A., Saxon, A. (1981). Pneumocytis carinii pneumonia and mucosal candidiasis in previously healthy homosexual men : Evidence of a new acquired cellular immuno-deficiency. N. Engl. J. Med. 305, 1426-1431.

Hahn, B.H., Shaw, G.M., Arya, S.U., Popovic, M., Gallo, R.C. and Wong-Stall, F. (1984). Molecular cloning and characterization of the HTLV-III virus associated with AIDS. Nature 312, 166-169.

Harris, J.D., Scott, J.V., Taylor, B., Brahic, M., Stowring, L., Ventura, P., Haase, A.T. and Peluso, R. (1981). Visna virus DNA : discovery of a novel gapped structure. Virology 113, 573-583.

Kiyokawa; T., Yoshikura, H., Hattori, S., Secki, M. and Yoshida, M. (1984). Envelope proteins of human T-cell leukemia virus : expression in Escherischia coli and its application to studies of env gene functions. Proc. Natl. Acad. Sci. USA 81, 6202-6206.

Klatzmann, D., Barré-Sinoussi, F., Nugeyre, M.T., Dauguet, C., Vilmer, E., Griscelli, C., Brun-Vézinet, F., RouziouxC., Gluckman, J.C., Chermann, J.C. and Montagnier, L. (1934). Selective tropism of lymphadenopathy associated virus (LAV) for helper-inducer T-lymphocytes. Sciences 223, 59-63.

Kozak, M. (1934). Compilation and analysis of sequences upstream from the transcriptional start site in eucaryotic mRNAs. Nucl. Acids Res. 12, 857-872.

Levy, J.A., Hoffman, A.D., Kramer, S.M., Lanois, J.A., Shimabukuro, J.M. and Oskiro, L.S. (1984). Isolation of lymphocytopathic retroviruses from San Francisco patients with AIDS. Science 225, 840-842.

Masur, H., Michelis, M.A., Greene, J.B., Onovato, I., Van de Stowe, R.A., Holzman, R.S., Wormser, G., Brettman, L., Lange, M., Murray, H.W., Cunningham-Rundles, S. (1981). An outbreak of community-acquired pneumocystis carinii pneumonia : Initial manifestation of cellular immune dysfunction. N. Engl. J. Med. 305, 1431-1438.

Misra, T.K., Grandgenett, D.P. and Parsons, J.T. (1982). Avian retrovirus pp32 DNA-binding protein. I. Recognition fo specific sequences on retrovirus DNA terminal repeats. J. Virol. 44, 330-343.

Montagnier, L., et al., (1984). A new human T-lymphotropic retrovirus : characterization and possible role in lymphadenopathy and acquired immune deficiency syndromes. In human T-cell leukemia/lymphoma viruses. R.C. Gallo, M. Essex and L. Gross, eds. (Cold Spring Laboratory, New-York), pp 363-370.

Oroszian, S., Copeland, T.D., Kalyanaraman, V.S., Sarngadharan, M.G., Schultz, A.M. and Gallo, R.C. (1984). Chemical analysis of human T-cell leukemia virus structural proteins. In HTLVS (R.C. Gallo, M.E. Essex and L. Gross, eds) Cold Spring Laboratory, New-York, pp 101-110.

Piot, P., Quinn, T.C., Taelmann, H., Feinsod, F.M. et al., (1984). Acquired immunodeficiency syndrome in a heterosexual population in Zaire. Lancet II, 65-69.

Popovic, M., Sarngadharan, M.G., Read, E. and Gallo, R.C. (1984). Detection, isolation, and continuous production of cytopathic retroviruses (HTLV-III) from patients with AIDS and pre-AIDS. Science 224, 497-300.

Querat, G., Barban, N., Sauze, N., Filippi, P., Vigne, R., Russo, P. and Vitu, C. (1984). Highly lytic and persistent lentiviruses naturally present in sheep with progressive pneumonia are genetically distinct. J. Virol. 52, 672-679.

Raba, M., Limburg, K., Burghagen, M., Katze, J.R., Simsek, M., Heckman, J.E., Rajbhandary, U.L., and Gross, H.J. (1979). Nucleotide sequence fo three isoaccepting lysine tRNAs from rabbit liver and SV40-transformed mouse fibroblasts. Eur. J. Biochem. 97, 303-318.

Rice, N.R., Stephens, R.M., Couez, D., Deschamps, J., Kettmann, R., Burny, A., and Gilden, R.V. (1984). The nucleotide sequence of the env gene and post-env region of bovine leukemia virus. Virology 138, 82-93.

Sagata, N., Yasunaga, T., Ogawa, Y., Tsuzuku-Kawamura, J. and Ikawa, Y. (1984). Bovine leukemia virus : Unique structural features of its long terminal repeats and its evolutionary relationship to human T-cell leukemia virus. Proc. Natl. Acad. Sci. USA 81, 4741-4745.

Sanger, F., Nicklen, S. and Coulsen, A.R. (1977). DNA sequencing with chain terminating inhibitors. Proc. Natl. Acad. Sci. USA 74, 5463-5467.

Schwartz, D.E., Tizard, R. and Gilbert, W. (1983). Nucleotide sequence of Rous sarcoma virus. Cell 32, 853-869.

Schüpbach, J., Popovic, M., Gilden, R.V., Gonda, M.A., Sarngadharan, M.G. and Gallo, R.C. (1984). Serological analysis of a subgroup of human T-lymphotropic retroviruses (HTLV-III) associated with AIDS. Science 224, 503-505.

Seiki, M., Hattori, S., Hirayama, Y. and Yoshida, M. (1983). Human adult T-cell leukemia virus : complete nucleotide sequence of the provirus genome integrated in leukemia cell DNA. Proc. Natl. Acad. Sci. USA 80, 3618-3622.

Shaw, G.M., Hahn, B.H., Arya, S.K., Groopman, J.E., Gallo, R.C. and Wong-Staal, F. (1984). Molecular characterization of human T-cell leukemia (lymphotropic) virus type III in the Acquired Immune Deficiency Syndrome. Science 226, 1163-1171.

Shimotohno, k., and Temin, H.M. (1982). Spontaneous variation and synthesis in the U3 region of the long terminal repeat of avion retroviruses. J. Virol. 41, 1636171.

Shimotohno, K., Golde, D.M., Miwa, M., Sugimura, T. and Chen, I.S.Y. (1984). Nucleotide sequence analysis to the long terminal repeat of human T-cell leukemia virus type II. Proc. Natl. Acad. Sci. USA 81, 1079-1083.

Shinnick, T.M., Lerner, R.A. and Sutcliffe, J.C. (1981). Nucleotide sequence of Moloney murine leukemia viruse. Nature 293, 543-548.

Srinivasan, A., Reddy, E.P., Dunn, C.Y. and Aaronson, S.A. (1984). Molecular dissection of transcriptional control elements with the long terminal repeat of retrovirus. Science 223, 286-289.

**Claims**

1. Recombinant or synthetic GAG antigen which consists in the amino acid sequence represented on figure 4a and 4b beginning with residue Met at position 336 and ending with residue Gln at position 1836.

2. Recombinant or synthetic GAG antigen according to claim 1 which is encoded by the Open Reading Frame corresponding to the nucleotide sequence comprised between nucleotide 336 and 1836 of the nucleotide sequence represented on figure 4.

3. Polypeptide fragment of an antigen according to anyone of claims 1 or 2, bearing an epitope of said antigen.

4. Polypeptide which distinguishes by one or several amino acids from a polypeptide according to anyone of claims 1 to 3, while having substantially the same immunological properties.

5. Polypeptide according to anyone of claims 1 to 4 which is recognized by antibodies contained in the serum of patients infected with LAV.

6. Polypeptide fragment according to claim 4 which is coupled to a physiologically acceptable and non toxic carrier molecule.

7. Polypeptide according to anyone of claims 1 to 6 which is purified from a LAV virus preparation.

8. A process for the preparation of a recombinant or synthetic core antigen designated p25, which comprises expressing in a cell an ORF sequence according to claim, 2 and obtaining a core antigen having an apparent molecular weight of 25000 Da by electrophoresis in SDS-polyacrylamide gel, and having the following NH2-terminal amino acid sequence :

    Pro Ile Val Gln Asn Ile Gln Gly Gln Met Val His Gln Ala Ile.

9. Process for the chemical synthesis of a peptide or polypeptide according to anyone of claims 1 to 7

**characterized by** :

- successively condensing either the successive aminoacids in twos, in the appropriate order or successive peptide fragments previously available or formed and containing already several aminoacyl residues in the appropriate order respectively, all the reactive groups other than the carboxyl and amino groups which will be engaged in the formation of the peptide bond being protected,
- possibly activating the carboxyl groups prior to the formation of the peptide bond,
- recovering the peptide or polypeptide formed.

10. Process for the preparation of the antigen according to anyone of claims 1 to 5, **characterized by** :

- the transformation of procaryotic or eucaryotic host with a suitable vector, previously modified by a DNA encoding a GAG antigen or a polypeptide fragment according to claims 1 to 5 in conditions enabling the production of the desired peptide,
- recovering and purifying the antigen thus obtained for instance electrophoresis.

11. Use of an antigen, a polypeptide or a polypeptide fragment according to anyone of claims 1 to 7 or use of a polypeptide obtained by a process of claim 8, for the in vitro detection of anti-LAV antibodies.

12. Use according to claim 11 wherein the detection is made by an ELISA technique

13. Kit for the detection of anti-LAV antibodies which comprises a polypeptide or a polypeptide fragment according to anyone of claims 1 to 7 and biological and chemical reagents and equipment necessary for performing diagnostic assays

14. A process for the detection of the presence of anti-LAV antibodies in a biological medium which comprises contacting said biological medium with an antigen, a polypeptide or a polypeptide fragment according to anyone of claims 1 to 7, or a polypeptide obtained by a process of claim 8, under conditions suitable for enabling a complex between said antibodies and said polypeptide to be formed, and detecting said complex as indicative of the presence of said antibodies in said biological fluid.

15. Use of antibodies recognizing specifically an antigen, a polypeptide or a polypeptide fragment according to anyone of claims 1 to 7 or a polypeptide obtained by a process of claim 8 for the identification thereof or even for the determination of relative proportion of this antigen, in a biological sample containing LAV or related virus.

16. Immunogenic composition **characterized in that** the protective principle is constituted by an antigen, a polypeptide or a polypeptide fragment according to anyone of claims 1 to 7 or a polypeptide obtained by a process of claim 8.

17. Process for the production of monoclonal antibodies against a determined antigen, a polypeptide or a polypeptide fragment according to anyone of claims 1 to 7 or a polypeptides obtained by a process of claim 8, comprising the steps of :

- fusing myeloma cells with splenocytes of an animal previously immunised with this antigen , to obtain hybridomas,
- selecting the hybridomas producing antibodies having specificity for antigen used for the immunisation,
- recovering the antibodies against the determined antigen.

**Patentansprüche**

1. Rekombinantes oder synthetisches GAG-Antigen, das aus der in Figur 4a und 4b dargestellten Aminosäuresequenz besteht, die mit dem Rest Met an Position 336 beginnt und mit dem Rest Gln an Position 1836 endet.

2. Rekombinantes oder synthetisches GAG-Antigen nach Anspruch 1, das von dem offenen Leserahmen codiert wird, der der Nucleotidsequenz entspricht, die zwischen Nucleotid 336 und 1836 der in Figur 4 dargestellten Nucleotidsequenz umfasst wird.

3. Polypeptidfragment eines Antigens nach einem der Ansprüche 1 oder 2, das ein Epitop des Antigens trägt.

**4.** Polypeptid, das sich durch ein oder mehrere Aminosäuren von einem Polypeptid nach einem der Ansprüche 1 bis 3 unterscheidet, wobei es im Wesentlichen die gleichen immunologischen Eigenschaften hat.

**5.** Polypeptid nach einem der Ansprüche 1 bis 4, das von Antikörpern erkannt wird, die im Serum von mit LAV infizierten Patienten enthalten sind.

**6.** Polypeptidfragment nach Anspruch 4, das mit einem physiologisch verträglichen und nicht toxischen Trägermolekül verknüpft ist.

**7.** Polypeptid nach einem der Ansprüche 1 bis 6, das aus einem LAV-Virus-Präparat gereinigt wird.

**8.** Verfahren zur Herstellung eines rekombinaten oder synthetischen Kernantigens, das als p25 bezeichnet wird, umfassend die Expression einer ORF-Sequenz nach Anspruch 2 in einer Zelle und das Erhalten eines Kernantigens, das ein bei einer Elektrophorese in SDS-Polyacrylamidgel offensichtliches Molekulargewicht von 25000 Da und die folgende $NH_2$-terminale Aminosäuresequenz hat:
Pro Ile Val Gln Asn Ile Gln Gly Gln Met Val His Gln Ala Ile.

**9.** Verfahren zur chemischen Synthese eines Peptids oder Polypeptids nach einem der Ansprüche 1 bis 7, **gekennzeichnet durch**:

- schrittweises Kondensieren entweder der aufeinanderfolgenden Aminosäuren in Zweiergruppen, in der richtigen Reihenfolge, oder von bereits vorhandenen bzw. gebildeten aufeinanderfolgenden Peptidfragmenten, die bereits verschiedene Aminoacylreste in der jeweils richtigen Reihenfolge enthalten, wobei alle reaktiven Gruppen außer den an der Bildung der Peptidbindung beteiligten Carboxyl- und Aminogruppen geschützt sind,
- möglicherweise Aktivieren der Carboxylgruppen vor der Bildung der Peptidbindung,
- Gewinnen des gebildeten Peptids oder Polypeptids.

**10.** Verfahren zur Herstellung des Antigens nach einem der Ansprüche 1 bis 5, **gekennzeichnet durch**:

- Transformation eines prokaryontischen oder eukaryontischen Wirts mit einem geeigneten Vektor, der zuvor **durch** eine DNA, die ein GAG-Antigen oder ein Polypeptidfragment nach den Ansprüchen 1 bis 9 codiert, unter Bedingungen modifiziert wurde, welche die Herstellung des gewünschten Peptids erlauben,
- Gewinnen und Reinigen des so erhaltenen Antigens, z. B. **durch** Elektrophorese.

**11.** Verwendung eines Antigens, eines Polypeptids oder eines Polypeptidfragments nach einem der Ansprüche 1 bis 7 oder Verwendung eines durch das Verfahren nach Anspruch 8 erhaltenen Polypeptids für den in vitro-Nachweis von Anti-LAV-Antikörpern.

**12.** Verwendung nach Anspruch 11, wobei der Nachweis durch ELISA-Verfahren erbracht wird.

**13.** Kit zum Nachweis von Anti-LAV-Antikörpern, das ein Polypeptid oder ein Polypeptidfragment nach einem der Ansprüche 1 bis 7 und biologische oder chemische Reagenzien und Mittel umfasst, die für die Durchführung diagnostischer Tests notwendig sind.

**14.** Verfahren zum Nachweis der Anwesenheit von Anti-LAV-Antikörpern in einem biologischen Medium, umfassend das Inkontaktbringen des biologischen Mediums mit einem Antigen, einem Polypeptid oder einem Polypeptidfragment nach einem der Ansprüche 1 bis 7, oder einem Polypeptid, das durch ein Verfahren nach Anspruch 8 erhalten wurde, unter Bedingungen, die für eine Komplexbildung zwischen den Antikörpern und dem herzustellenden Polypeptid geeignet sind, und Nachweisen des Komplexes als Hinweis auf die Anwesenheit von Antikörpern in der biologischen Flüssigkeit.

**15.** Verwendung der Antikörper, die ein Antigen, ein Polypeptid oder ein Polypeptidfragment nach einem der Ansprüche 1 bis 7 oder ein durch ein Verfahren nach Anspruch 8 erhaltenes Polypeptid spezifisch erkennen, für deren Identifizierung oder auch zur Bestimmung des relativen Anteils des Antigens in einer biologischen Probe, die LAV oder ein verwandtes Virus enthält.

**16.** Immunogene Zusammensetzung, **dadurch gekennzeichnet, dass** das schützende Prinzip durch ein Antigen, ein Polypeptid oder ein Polypeptidfragment nach einem der Ansprüche 1 bis 7 oder ein durch ein Verfahren nach Anspruch 8 erhaltenes Polypeptid gewährleistet wird.

**17.** Verfahren zur Herstellung von monoclonalen Antikörpern gegen ein bestimmtes Antigen, ein Polypeptid oder ein Polypeptidfragment nach einem der Ansprüche 1 bis 7 oder ein durch ein Verfahren nach Anspruch 8 erhaltenes Polypeptid, umfassend die Schritte:

- Fusionieren von Myelomzellen mit Splenocyten eines zuvor mit diesem Antigen immunisierten Tiers, um Hybridome zu erhalten,
- Auswählen der Hybridome, welche Antikörper mit Spezifität für das bei der Immunisierung verwendete Antigen haben,
- Gewinnen der Antikörper gegen das bestimmte Antigen.

**Revendications**

**1.** Antigène GAG recombinant ou synthétique qui consiste en la séquence d'acides aminés représentée sur les figures 4a et 4b commençant par le résidu Met à la position 336 et se terminant par le résidu Gln à la position 1836.

**2.** Antigène GAG recombinant ou synthétique selon la revendication 1 qui est codé par le cadre ouvert de lecture correspondant à la séquence nucléotidique comprise entre les nucléotides 336 et 1836 de la séquence nucléotidique représentée sur la figure 4.

**3.** Fragment polypeptidique d'un antigène selon l'une quelconque des revendications 1 ou 2, portant un épitope dudit antigène.

**4.** Polypeptide qui se différencie par un ou plusieurs acides aminés d'un polypeptide selon l'une quelconque des revendications 1 à 3, tout en ayant sensiblement les mêmes propriétés immunologiques.

**5.** Polypeptide selon l'une quelconque des revendications 1 à 4 qui est reconnu par des anticorps contenus dans le sérum de patients infectés par le LAV.

**6.** Fragment polypeptidique selon la revendication 4 qui est couplé à une molécule de transport physiologiquement acceptable et non toxique.

**7.** Polypeptide selon l'une quelconque des revendications 1 à 6 qui est purifié à partir d'une préparation de virus LAV.

**8.** Procédé pour la préparation d'un antigène core recombinant ou synthétique désigné p25, qui comprend l'expression dans une cellule d'une séquence d'ORF selon la revendication 2, et l'obtention d'un antigène du noyau (core) possédant un poids moléculaire apparent de 25 000 Da par électrophorèse sur gel de SDS-polyacrylamide, et ayant la séquence d'acides aminés $NH_2$-terminale suivante :
Pro Ile Val Gln Asn Ile Gln Gly Gln Met Val His Gln Ala Ile.

**9.** Procédé pour la synthèse chimique d'un peptide ou d'un polypeptide selon l'une quelconque des revendications 1 à 7, **caractérisé par** :

- la condensation successive soit d'aminoacides successifs par deux, dans l'ordre approprié, soit de fragments peptidiques successifs préalablement disponibles ou formés et contenant déjà plusieurs résidus aminoacyle dans l'ordre approprié, respectivement, tous les groupes réactifs autres que les groupes carboxyle et amino qui seront engagés dans la formation de la liaison peptidique étant protégés,
- l'activation possible des groupes carboxyle avant la formation de la liaison peptidique,
- la récupération du peptide ou du polypeptide formé.

**10.** Procédé pour la préparation de l'antigène selon l'une quelconque des revendications 1 à 5, **caractérisé par** :

- la transformation d'un hôte procaryote ou eucaryote avec un vecteur adapté, préalablement modifié avec un ADN codant pour un antigène GAG ou un fragment polypeptidique selon les revendications 1 à 5, dans des conditions permettant la production du peptide désiré,
- la récupération et la purification de l'antigène ainsi obtenu, par exemple par électrophorèse.

**11.** Utilisation d'un antigène, d'un polypeptide ou d'un fragment polypeptidique selon l'une quelconque des revendications 1 à 7 ou utilisation d'un polypeptide obtenu par un procédé selon la revendication 8 pour la détection *in vitro* d'anticorps anti-LAV.

**12.** Utilisation selon la revendication 11 dans laquelle la détection est réalisée par une technique ELISA.

**13.** Kit pour la détection d'anticorps anti-LAV qui comprend un polypeptide ou un fragment polypeptidique selon l'une quelconque des revendications 1 à 7 et les réactifs biologiques et chimiques et l'équipement nécessaires pour effectuer des dosages de diagnostic.

**14.** Procédé pour la détection de la présence d'anticorps anti-LAV dans un milieu biologique qui comprend la mise en contact dudit milieu biologique avec un antigène, un polypeptide ou un fragment polypeptidique selon l'une quelconque des revendications 1 à 7, ou un polypeptide obtenu par un procédé selon la revendication 8, dans des conditions adaptées pour permettre qu'un complexe entre lesdits anticorps et ledit polypeptide se forme, et la détection dudit complexe comme indicateur de la présence desdits anticorps dans ledit fluide biologique.

**15.** Utilisation d'anticorps reconnaissant spécifiquement un antigène, un polypeptide ou un fragment polypeptidique selon l'une quelconque des revendications 1 à 7, ou un polypeptide obtenu par un procédé selon la revendication 8, pour l'identification de celui-ci ou même pour la détermination de la proportion relative de cet antigène, dans un échantillon biologique contenant le LAV ou un virus apparenté.

**16.** Composition immunogène **caractérisée en ce que** le principe protecteur est constitué par un antigène, un polypeptide ou un fragment polypeptidique selon l'une quelconque des revendications 1 à 7, ou un polypeptide obtenu par un procédé selon la revendication 8.

**17.** Procédé pour la production d'anticorps monoclonaux dirigés contre un antigène, un polypeptide ou un fragment polypeptidique déterminé selon l'une quelconque des revendications 1 à 7, ou un polypeptide déterminé obtenu par un procédé selon la revendication 8, comprenant les étapes de :

   - fusion de cellules de myélome avec des splénocytes d'un animal préalablement immunisé avec cet antigène, afin d'obtenir des hybridomes,
   - sélection des hybridomes produisant des anticorps ayant une spécificité pour l'antigène utilisé pour l'immunisation,
   - récupération des anticorps dirigés contre l'antigène déterminé.

FIG.1

FIG. 2

H=HindⅢ     K=Kpn I
S=Sac I       R=EcoRI
B= Bam HI    Sa=Sal I
P=Pst I        X=Xho I
Bg=Bgl Ⅱ

FIG. 3

Hind III

GGTCTCTCTGGTTAGACCAGATTTGAGCCTGGGAGCTCTCTCGCCTAACTAGGCAACCCACTGCTTAAGCCTCAATAAAGCTTGCCTTGAGTGCTTCAAGTAGTGTGTCCCCGTCTGTTCT
                                                                                                    100

GTGACTCTGGTAACTAGAGATCCCTCAGACCCTTTTAGTCAGTGTCGAAAATCTCTAGCAGTGGCCGCCCCGAACAGGGACTTGAAAGCCGAAAGGGAAACCAGAGGACCTCTCTCGACGCAG
                                                                                                    200

                                                                    GAG    LeuAlaGluAlaArgArgArgGluMetGlyAlaArgAlaSerValLeuSer
GACTCGGCTTGCTGAAGCGCCCACGGCAAGAGGCGAGCGGGAGCCGACTCGTGAGTACGCCAAAAAATTTTGACTAGCGGAGGCTAGAAGGAGAGAGATCGGTGCCGACAGCCGTCAGTATTAA
                                                                                                    300

  GlyGlyGluLeuAspArgTrpGluLysIleArgLeuArgProGlyGlyLysLysLysTyrLysLeuLysHisIleValTrpAlaSerArgGluLeuGluArgPheAlaValAsnProGly
GCGCGCGGAGAATTAGATCGATGGAAAAAATTCCGTTAAGCCCAGCGGGCAAAGAAAAAATATAAATTAAAACATATAGTATCGGCAAGCAGGCGAGCTAGAACGATTCGCAGTTAATCCTG
                                                                                                    400

  LeuLeuGluThrSerGluGlyCysArgGlnIleLeuGlyGlnLeuGlnProSerLeuGlnThrGlySerGluGluLeuArgSerLeuTyrAsnThrValAlaThrLeuTyrCysValHis
GCCTGTTAGAAACATCAGAAGGCTGTAGACAAATACTGGGACAGCTACAACCATCCCTTCAGACAGGATCAGAAGAACTTAGATCATTATATAATACAGTAGCAACCCTCTATTGTGTGCC
         500                                                                                        600

  GlnArgIleGluIleLysAspThrLysGluAlaLeuAspLysIleGluGluGluGlnAsnLysSerLysLysLysAlaGlnGlnAlaAlaAlaAspThrGlyHisSerSerGlnValSer
ATCAAAGGATAGAGATAAAAGACACCAAGGAAGCTTTAGACAAGATAGAGGAAGAGCAAAACAAAAGTAAGAAAAAAGCACAGCAAGCAGCAGCTGACACAGGACACAGCAGCCAGGTCA
                                                                                                    700

  GlnAspTyr ProIleValGlnAsnIleGlnGlyGlnMetValHisGlnAlaIle SerProArgThrLeuAsnAlaTrpValLysValValGluGluLysAlaPheSerProGluValIle
GCCAAAATTACCCTATAGTGCAGAACATCCAGGGGCAAATGGTACATCAGGCCATATCACCTAGAACTTTAAATGCATGGGTAAAAGTAGTAGAAGAGAAGGCTTTCACCCCAGAAGTGA
                                                                                                    800

  ProMetPheSerAlaLeuSerGluGlyAlaThrProGlnAspLeuAsnThrMetLeuAsnThrValGlyGlyHisGlnAlaAlaMetGlnMetLeuLysGluThrIleAsnGluGluAla
TACCCATGTTTTTCAGCATTATCAGAAGGAGCCACCCCACAAGATTTAAACACCATGCTAAACACAGTGGGGGGACATCAAGCAGCCATGCAAATGTTAAAAGAGACCATCAATGAGGAAG
                                                                                                    900

  AlaGluTrpAspArgValHisProValHisAlaGlyProIleAlaProGlyGlnMetArgGluProArgGlySerAspIleAlaGlyThrThrSerThrLeuGlnGluGlnIleGlyTrp
CTGCAGAATGGGATAGAGTGGATCCAGTGCATGCAGGGCCTATTGCACCAGGCCAGATGAGAGAACCAAGGGGAAGTGACATAGCAGGAACTACTAGTACCCTTCAGGAACAAATAGGAT
                                                                                                    1000

  MetThrAsnAsnProProIleProValGlyGluIleTyrLysArgTrpIleIleLeuGlyLeuAsnLysIleValArgMetTyrSerProThrSerIleLeuAspIleArgGlnGlyPro
GGATGACAAATAATCCACCTATCCCAGTAGGAGAAATTTATAAAAGATGGATAATCCTGGGATTAAATAAAATAGTAAGAATGTATAGCCCTACCAGCATTCTGGACATAAGACAAGGAC
  1100                                                                                             1200

  LysGluProPheArgAspTyrValAspArgPheTyrLysThrLeuArgAlaGluGlnAlaSerGlnGluValLysAsnTrpMetThrGluThrLeuLeuValGlnAsnAlaAsnProAsp
CAAAAGAACCCTTTAGAGACTATGTAGACCGGTTCTATAAAACTCTAAGAGCCGAGCAAGCTTCACAGGAGGTAAAAAATTGGATGACAGAAACCTTGTTGGTCCAAAATGCGAACCCAG
                                                                                                    1300

  CysLysThrIleLeuLysAlaLeuGlyProAlaAlaThrLeuGluGluMetMetThrAlaCysGlnGlyValGlyGlyProGlyHisLysAlaArgValLeuAlaGluAlaMetSerGln
ATTGTAAGACTATTTTAAAAGCATTGGGACCAGCAGCTACACTAGAAGAAATGATGACAGCATGTCAGGGAGTCGGACGACCCCGCCATAAGCCAAGAGTTTTTGGCTGAAGCAATGAGCC
                                                                                                    1400

FIG. 4A

ValThrAsnSerAlaThrIleMetMetGlnArgGlyAsnPheArgAsnGlnArgLysIleValLysCysPheAsnCysGlyLysGluGlyHisIleAlaArgAsnCysArgAlaProArg
AAGTAACAAATTCAGCTACCATAATGATGCAAAGAGCCAATTTTAGGAACCAAAGAAAGATTGTTAAGTGTTTCAATTGTGGCAAAGAAGGGCACATAGCCAGAAATTGCAGGGCCCCTA

                                                          .1500
                                                POL ⇒ PhePheArgGluAspLeuAlaPheLeuGlnGlyLysAlaArgGluPheSer
LysLysGlyCysTrpLysCysGlyLysGluGlyHisGlnMetLysAspCysThrGluArgGlnAlaAsnPheLeuGlyLysIleTrpProSerTyrLysGlyArgProGlyAsnPheLeu
GGAAAAAGGGCTGTTGGAAATGTGGAAAGGAAGGACACCCAAATGAAAGATTGTACTGAGAGACAGGCTAATTTTTTAGGGAAGATCTGGCCTTCCTACAAGGGAAGGCCAGCGAATTTTC

                    1600
SerGluGlnThrArgAlaAsnSerProThrArgArgGluLeuGlnValTrpGlyArgAspAsnAsnSerLeuSerGluAlaGluAlaAspArgGlnGlyThrValSerPheAsnPhePro
GlnSerArgProGluProThrAlaProProGluGluSerPheArgSerGlyValGluThrThrThrProSerGlnLysGlnGluProIleAspLysGluLeuTyrProLeuThrSerLeu
TTCAGAGCCAGACCAGAGCCCAACAGCCCCACCAGAAGAGAGCCTTCAGGTCTCGCGGTAGAGACAACAACTCCCTCTCAGAAGCAGGAGCCGATAGACAAGGAACTGTATCCTTTAACTTCCC

                    1700                                                                                      1800
GlnIleThrLeuTrpGlnArgProLeuValThrIleLysIleGlyGlyGlnLeuLysGluAlaLeuLeuAspThrGlyAlaAspAspThrValLeuGluGluMetSerLeuProGlyArg
ArgSerLeuPheGlyAsnAspProSerSerGln *
TCAGATCACTCTTTGGCAACGACCCCTCGTCACAATAAAGATAGGGGGGCAACTAAAGGAAGCTCTATTAGATACAGGAGCAGATGATACAGTATTAGAAGAAATGAGTTTGCCAGGAAG

                                                                  1900
TrpLysProLysMetIleGlyGlyIleGlyGlyPheIleLysValArgGlnTyrAspGlnIleLeuIleGluIleCysGlyHisLysAlaIleGlyThrValLeuValGlyProThrPro
ATGGAAACCAAAAATGATAGGGCGAATTCGAGGTTTTATCAAAGTAAGACAGTATGATCAGATACTCATACAAATCTGTGGACATAAAGCTATAGGTACAGTATTAGTAGGACCTACACC

                                                        2000
ValAsnIleIleGlyArgAsnLeuLeuThrGlnIleGlyCysThrLeuAsnPheProIleSerProIleGluThrValProValLysLeuLysProGlyMetAspGlyProLysValLys
TGTCAACATAATTCGAAGAAATCTCTTGACTCAGATTGGTTGCACTTTAAATTTTCCCATTAGTCCTATTGAAACTGTACCAGTAAAATTAAAGCCAGGAATGGATCGCCCAAAGTTAA

                        2100
GlnTrpProLeuThrGluGluLysIleLysAlaLeuValGluIleCysThrGluMetGluLysGluGlyLysIleSerLysIleGlyProGluAsnProTyrAsnThrProValPheAla
ACAATCGCCATTGACAGAAGAAAAAATAAAAGCATTAGTAGAAATTTGTACAGAAATGGAAAAGGAAGCGAAAATTTCAAAAATTCGGCCTGAAAATCCATACAATACTCCAGTATTTGC

                        2200
IleLysLysLysAspSerThrLysTrpArgLysLeuValAspPheArgGluLeuAsnLysArgThrGlnAspPheTrpGluValGlnLeuGlyIleProHisProAlaGlyLeuLysLys
CATAAAGAAAAAAGACAGTACTAAATGGAGAAAATTAGTAGATTTCAGAGAACTTAATAAGAGAACTCAAGACTTCTGCGAAGTTCAATTAGGAATACCACATCCCGCAGGGTTAAAAAA

                        2300                                                              2400
LysLysSerValThrValLeuAspValGlyAspAlaTyrPheSerValProLeuAspGluAspPheArgLysTyrThrAlaPheThrIleProSerIleAsnAsnGluThrProGlyIle
GAAAAAATCAGTAACAGTACTGGATGTGGGTGATGCATATTTTTCAGTTCCCTTAGATGAAGACTTCAGGAAGTATACTGCATTTACCATACCTAGTATAAACAATGAGACACCAGGGAT

                                                                        2500
ArgTyrGlnTyrAsnValLeuProGlnGlyTrpLysGlySerProAlaIlePheGlnSerSerMetThrLysIleLeuGluProPheArgLysGlnAsnProAspIleValIleTyrGln
TAGATATCAGTACAATGTGCTTCCACAGGGATGGAAAGGATCACCAGCAATATTCCAAAGTAGCATGACAAAAATCTTAGAGCCTTTTAGAAAACAAAATCCAGACATAGTTATCTATCA

                                        2600

**FIG. 4B**

TyrMetAspAspLeuTyrValGlySerAspLeuGluIleGlyGlnHisArgThrLysIleGluGluLeuArgGlnHisLeuLeuArgTrpGlyLeuThrThrProAspLysLysHisGln
ATACATCGATGATTTCTATCTAGGATCTGACTTAGAAATACGGCACCATAGAACAAAAATAGACGAGCTCGAGACAACATCTCTTGACGTGCCGACTTACCACACCAGACAAAAAACATCA

2700

LysGluProProPheLeuTrpMetGlyTyrGluLeuHisProAspLysTrpThrValGlnProIleValLeuProGluLysAspSerTrpThrValAsnAspIleGlnLysLeuValGly
GAAAGAACCTCCATTCCTTTCGATGGGTTATGAACTCCATCCTGATAAATCGACAGTACAGCCTATAGTGCTGCCAGAAAAAGACAGCTCGACTGTCAATCACATACAGAAGTTAGTCGCG

2800

LysLeuAsnTrpAlaSerGlnIleTyrProGlyIleLysValArgGlnLeuCysLysLeuLeuArgGlyThrLysAlaLeuThrGluValIleProLeuThrGluGluAlaGluLeuGlu
AAAATTGAATTGGGCAAGTCAGATTTACCCACGGATTAAAGTAAGGCAATTATGTAAACTCCTTAGACGAACCAAAGCACTAACAGAAGTAATACCACTAACAGAAGAAGCAGAGCTAGA

2900                                                                                                                    3000

LeuAlaGluAsnArgGluIleLeuLysGluProValHisGlyValTyrTyrAspProSerLysAspLeuIleAlaGluIleGlnLysGlnGlyGlnGlyGlnTrpThrTyrGlnIleTyr
ACTCGGCAGAAAACAGAGAGATTCTAAAAGAACCAGTACATCGAGTGTATTATGACCCATCAAAAGACTTAATAGCAGAAATACAGAAGCACGGGCAAGCCCAATGGACATATCAAATTTA

3100

GlnGluProPheLysAsnLeuLysThrGlyLysTyrAlaArgThrArgGlyAlaHisThrAsnAspValLysGlnLeuThrGluAlaValGlnLysIleThrThrGluSerIleValIle
TCAAGAGCCATTTAAAAATCTGAAAACAGGAAAATATCCAACAACGAGGGGTGCCCACACTAATCATGTAAAACAATTAACAGAGGCAGTGCCAAAAAATAACCACAGAAAGCATAGTAAT

3200

TrpGlyLysThrProLysPheLysLeuProIleGlnLysGluThrTrpGluThrTrpTrpThrGluTyrTrpGlnAlaThrTrpIleProGluTrpGluPheValAsnThrProProLeu
ATGCGGAAAGACTCCTAAATTTAAACTACCCATACAAAAGGAAACATGGGAAACATCGTGGACAGAGTATTGGCAAGCCACCTGGATTCCTGAGTGGGAGTTTGTCAATACCCCTCCTTT

3300

ValLysLeuTrpTyrGlnLeuGluLysGluProIleValGlyAlaGluThrPheTyrValAspGlyAlaAlaSerArgGluThrLysLeuGlyLysAlaGlyTyrValThrAsnArgGly
AGTGAAATTATCGTACCAGTTAGACAAACAACCCATAGTACGGAGCAGAAACGTTCTATCTAGATGGGGCAGCTAGCAGGGAGACTAAATTAGGAAAAGCAGGATATGTTACTAATAGAGG

3400

ArgGlnLysValValThrLeuThrAspThrThrAsnGlnLysThrGluLeuGlnAlaIleHisLeuAlaLeuGlnAspSerGlyLeuGluValAsnIleValThrAspSerGlnTyrAla
AAGACAAAAAGTTGTCACCCTAACTGACACAACAAATCAGAAGACTGAGTTACAAGCAATTCATCTAGCTTTGCAGGATTCGGGATTAGAAGTAAATATAGTAACAGACTCACAATATGC

3500                                                                                                                    3600

LeuGlyIleIleGlnAlaGlnProAspLysSerGluSerGluLeuValAsnGlnIleIleGluGlnLeuIleLysLysGluLysValTyrLeuAlaTrpValProAlaHisLysGlyIle
ATTAGGAATCATTCAAGCACAACCAGATAAAAGTGAATCAGAGTTAGTCAATCAAATAATAGAGCAGTTAATAAAAAAGGAAAAGGTCTATCTGGCATGGGTACCAGCACACAAAGGAAT

3700

GlyGlyAsnGluGlnValAspLysLeuValSerAlaGlyIleArgLysValLeuPheLeuAspGlyIleAspLysAlaGlnAspGluHisGluLysTyrHisSerAsnTrpArgAlaMet
TCGACGAAATGAACAAGTAGATAAATTAGTCAGTGCTGGAATCAGGAAAGTACTATTTTTAGATGGAATAGATAAGGCCCAAGATGAACATGAGAAATATCACAGTAATTCGAGAGCAAT

3800

AlaSerAspPheAsnLeuProProValValAlaLysGluIleValAlaSerCysAspLysCysGlnLeuLysGlyGluAlaMetHisGlyGlnValAspCysSerProGlyIleTrpGln
GGCTAGTGATTTTAACCTGCCACCTGTAGTAGCAAAAGAAATAGTAGCCAGCTGTGATAAATGTCAGCTAAAAGGAGAAGCCATGCATGGACAAGTAGACTGTAGTCCAGGAATATGGCA

3900

FIG. 4C

LeuAspCysThrHisLeuGluGlyLysValIleLeuValAlaValHisValAlaSerGlyTyrIleGluAlaGluValIleProAlaGluThrGlyGlnGluThrAlaTyrPheLeuLeu
ACTAGATTGTACACATTTAGAAGGAAAAGTTATCCTGGTACCACTTCATGTAGCCAGTGGATATATAGAAGCAGAAGTTATTCCAGCCAGAAACAGGGCAGGAAACAGCATACTTTCTTTT
    4000

LysLeuAlaGlyArgTrpProValLysThrIleHisThrAspAsnGlySerAsnPheThrSerThrThrValLysAlaAlaCysTrpTrpAlaGlyIleLysGlnGluPheGlyIlePro
AAAATTAGCAGGAAGATCGCCAGTAAAAACAATACATACAGACAATGGCAGCAATTTCACCAGTACTACGGTTAAGGCCGCCTGTTGGTGGCCGGGAATCAAGCAGGAATTTCGGAATTCC
    4100    4200

TyrAsnProGlnSerGlnGlyValValGluSerMetAsnLysGluLeuLysLysIleIleGlyGlnValArgAspGlnAlaGluHisLeuLysThrAlaValGlnMetAlaValPheIle
CTACAATCCCCAAAGTCAAGGAGTAGTAGAATCTATGAATAAAGAATTAAAGAAAATTATACGCCAGGTAAGAGATCAGGCTGAACATCTTAAGACAGCAGTACAAATGGCAGTATTCAT
    4300

HisAsnPheLysArgLysGlyGlyIleGlyGlyTyrSerAlaGlyGluArgIleValAspIleIleAlaThrAspIleGlnThrLysGluLeuGlnLysGlnIleThrLysIleGlnAsn
CCACAATTTTAAAAGAAAACGGGCGGATTGGCCGGGTACAGTGCCAGGGGCAAAGAATAGTAGACATAATAGCAACAGACATACAAACTAAAGAATTACAAAAACAAATTACAAAAATTCAAAA
    4400

PheArgValTyrTyrArgAspSerArgAspProLeuTrpLysGlyProAlaLysLeuLeuTrpLysGlyGluGlyAlaValValIleGlnAspAsnSerAspIleLysValValProArg
    ORF Q  CysGlnGlu
TTTTCGGGTTTATTACAGGGACAGCAGAGATCCACTTTGGAAACGACCAGCAAAGCTCCTCTGGAAACGTGAACGGGCAGTAGTAATACAAGATAATAGTGACATAAAAGTAGTCCCAAG
    4500

ArgLysAlaLysIleIleArgAspTyrGlyLysGlnMetAlaGlyAspAspCysValAlaSerArgGlnAspGluAsp *
GluLysGlnArgSerLeuGlyIleMetGluAsnArgTrpGlnValMetIleValTrpGlnValAspArgMetArgIleArgThrTrpLysSerLeuValLysHisHisMetTyrValSer
AAGAAAAGCAAAGATCATTAGGGATTATGGAAAACAGATGGCAGGTGATGATTGTGTGGCAAGTAGACAGGATGAGGATTAGAACATGGAAAAGTTTAGTAAAACACCATATGTATGTTT
    4600

GlyLysAlaArgGlyTrpPheTyrArgHisHisTyrGluSerProHisProArgIleSerSerGluValHisIleProLeuGlyAspAlaArgLeuValIleThrThrTyrTrpGlyLeu
CACGGGAAAGCTAGGGGATGGTTTTATAGACATCACTATGAAAGCCCTCATCCAAGAATAAGTTCAGAAGTACACATCCCACTAGGGGATGCTAGCATTCGTAATAACAACATATTGGGGTC
    4700    4800

HisThrGlyGluArgAspTrpHisLeuGlyGlnGlyValSerIleGluTrpArgLysLysArgTyrSerThrGlnValAspProGluLeuAlaAspGlnLeuIleHisLeuTyrTyrPhe
TGCATACACGAGAAAGAGACTCGCCATCTGGGTCACGGAGTCTCCATAGAATGGAGGAAAAAGAGATATAGCACACAAGTAGACCCTGAACTAGCAGACCAACTAATTCATCTGTATTACT
    4900

AspCysPheSerAspSerAlaIleArgLysAlaLeuLeuGlyHisIleValSerProArgCysGluTyrGlnAlaGlyHisAsnLysValGlySerLeuGlnTyrLeuAlaLeuAlaAla
TTGACTGTTTTTCAGACTCTGCTATAAGAAAGGCCTTATTAGGACATATAGTTAGCCCTAGGTGTGAATATCAAGCACGACATAACAAGGTAGGATCTCTACAATACTTGGCACTAGCAG
    5000

LeuIleThrProLysLysIleLysProProLeuProSerValThrLysLeuThrGluAspArgTrpAsnLysProGlnLysThrLysGlyHisArgGlySerHisThrMetAsnGlyHis
CATTAATAACACCAAAAAAGATAAAGCCCACCTTTGCCTAGTGTTACGAAACTGACAGAGGATAGATGGAACAAGCCCCAGAAGACCAAGGGCCACAGAGGGAGCCACACAATGAATCGAC
    5100

FIG. 4D

ACTAGAGCTTTTAGAGGAGCTTAAGAATGAAGCTGTTAGACATTTTCCTAGGATTTGGCTCCATCGCTTAGGGCAACATATCTATGAAACTTATCGGGATACTTGCGCAGGAGTGGAAGC
.               5200                .                                          .                                .

CATAATAAGAATTCTGCAACAACTGCTGTTTATCCATTTCAGAATTGGGTGTCGACATAGCCAGAATAGCCGTTACTCAACAGACGAGAGCAAGAAATGGAGCCAGTAGATCCTAGACTAG
.          5300           .        .   .                    .                           .             .                .   5400

AGCCCTGGAAGCATCCAGGAAGTCAGCCTAAAACTGCTTGTACCACTTGCTATTGTAAAAAGTGTTGCTTTCATTGCCAAGTTTGTTTCACAACAAAAGCCTTAGGCATCTCCTATGGCA
.                          .          .   .    .                           .                      .    5500              .

GGAAGAAGCGGAGACACGCGACGAAGACCTCCTCAAGGCAGTCAGACTCATCAAGTTTCTCTATCAAAGCAGTAAGTAGTACATGTAATGCAACCTATACAAATACCAATAGCAGCATTAG
.              .               .          .               .                     .   5600   .          .

                                                                                                    EBV → LysGluGlnLysThr
TAGTAGCAATAATAATAGCAATAGTTGTGTGGTCCATAGTAATCATAGAATATAGGAAAATATTAAGACAAAGAAAAATAGACAGGTTAATTGATAGACTAATAGAAAGAGCAGAAGACA
.                            .                      .     5700    .                   .                 .              .

ValAlaMetArgValLysGluLysTyrGlnHisLeuTrpArgTrpGlyTrpLysTrpGlyThrMetLeuLeuGlyIleLeuMetIleCysSerAlaThrGluLysLeuTrpValThrVal
GTGGCAATGAGACTGAAGGAGAAATATCACCACTTGTGGAGATGGGGGTGGAAATGGGGCACCATCCTCCTTCGGATATTGATGATCTGTAGTGCTACAGAAAAATTGTGGGTCACAGTC
.            .              5800         .              .                 .                       .

TyrTyrGlyValProValTrpLysGluAlaThrThrThrLeuPheCysAlaSerAspAlaLysAlaTyrAspThrGluValHisAsnValTrpAlaThrHisAlaCysValProThrAsp
TATTATGGGGTACCTGTGTGCAAGGAAGCAACCACCACTCTATTTTGTGCATCAGATGCTAAAGCATATGATACAGACGTACATAATGTTTGGGCCACACATGCCTGTGTACCCACAGAC
.              5900        .          .                   .                .                       .        .   6000

ProAsnProGlnGluValValLeuValAsnValThrGluAsnPheAsnMetTrpLysAsnAspMetValGluGlnMetHisGluAspIleIleSerLeuTrpAspGlnSerLeuLysPro
CCCAACCCACAAGAAGTAGTATTGGTAAATGTGACAGAAAATTTTAACATGTGGAAAAATGACATGGTAGAACAGATGCATGAGGATATAATCAGTTTATGGGATCAAAGCCTAAAGCCA
.                 .                .                       .                 .                      .    6100    .

CysValLysLeuThrProLeuCysValSerLeuLysCysThrAspLeuGlyAsnAlaThrAsnThrAsnSerSerAsnThrAsnSerSerSerGlyGluMetMetMetGluLysGlyGlu
TGTGTAAAATTAACCCCACTCTGTGTTAGTTTAAAGTGCACTGATTTCGGGAATGCTACTAATACCAATAGTAGTAATACCAATAGTAGTAGCGGCGAAATGATGATGGAGAAAGGAGAG
.                    .                  .                         .           6200          .                .

IleLysAsnCysSerPheAsnIleSerThrSerIleArgGlyLysValGlnLysGluTyrAlaPhePheTyrLysLeuAspIleIleProIleAspAsnAspThrThrSerTyrThrLeu
ATAAAAAACTGCTCTTTCAATATCAGGACAAGCATAAGAGGTAAGGTGCAGAAAGAATATGCATTTTTTTATAAACTTGATATAATACCAATAGATAATGATACTACCAGCTATACGTTG
.                .                .                          .               6300

ThrSerCysAsnThrSerValIleThrGlnAlaCysProLysValSerPheGluProIleProIleHisTyrCysAlaProAlaGlyPheAlaIleLeuLysCysAsnAsnLysThrPhe
ACAAGTTGTAACACCTCAGTCATTACACAGCCCTGTCCAAAGGTATCCTTTGAGCCAATTCCCATACATTATTGTGCCCCGGCTGGTTTTGCGATTCTAAAATGTAATAATAAGACGTTC
.                .                6400         .                          .

AsnGlyThrGlyProCysThrAsnValSerThrValGlnCysThrHisGlyIleArgProValValSerThrGlnLeuLeuLeuAsnGlySerLeuAlaGluGluGluValValIleArg
AATGGAACAGGACCATGTACAAATGTCAGCACAGTACAATGTACACATGGAATTAGGCCAGTAGTATCAACTCAACTGCTGTTGAATGGCAGTCTAGCAGAAGAAGAGGTAGTAATTAGA
.              6500            .              .                .           .              .                      6600

FIG. 4E

SerAlaAsnPheThrAspAsnAlaLysThrIleIleValGlnLeuAsnGlnSerValGluIleAsnCysThrArgProAsnAsnAsnThrArgLysSerIleArgIleGlnArgGlyPro
TCTGCCAATTTCACAGACAATGCTAAAACCATAATAGTACAGCTGAACCAATCTGTAGAAATTAATTGTACAAGACCCAACAACAATACAAGAAAAAGTATCCGTATCCAGAGCGGACCA
6700

GlyArgAlaPheValThrIleGlyLysIleGlyAsnMetArgGlnAlaHisCysAsnIleSerArgAlaLysTrpAsnAlaThrLeuLysGlnIleAlaSerLysLeuArgGluGlnPhe
GGGAGAGCCATTTGTTACAATAGGAAAAATAGGAAATATGAGACAAGCACATTGTAACATTAGTAGAGCCAAAATGGAATGCCAGTTTAAAACAGATAGCTAGCAAATTAAGAGAACAATTT
6800

GlyAsnAsnLysThrIleIlePheLysGlnSerSerGlyGlyAspProGluIleValThrHisSerPheAsnCysGlyGlyGluPhePheTyrCysAsnSerThrGlnLeuPheAsnSer
GGAAATAATAAAACAATAATCTTTAAGCAATCCTCAGGAGGGGACCCAGAAATTGTAACGCACAGTTTTAATTGTGGACGGGAATTTTTCTACTGTAATTCAACACAACTGTTTAATAGT
6900

ThrTrpPheAsnSerThrTrpSerThrGluGlySerAsnAsnThrGluGlySerAspThrIleThrLeuProCysArgIleLysGlnPheIleAsnMetTrpGlnGluValGlyLysAla
ACTTGGTTTAATAGTACTTGGAGTACTGAAGGGTCAAATAACACTGAAGGAAGTGACACAATCACACTCCCATGCAGAATAAAACAATTTATAAACATGTGGCAGGAAGTAGGAAAAGCA
7000

MetTyrAlaProProIleSerGlyGlnIleArgCysSerSerAsnIleThrGlyLeuLeuLeuThrArgAspGlyGlyAsnAsnAsnAsnGlySerGluIlePheArgProGlyGlyGly
ATGTATGCCCCTCCCATCAGCCGGACAAATTAGATGTTCATCAAATATTACAGGGCTGCTATTAACAAGAGATGGTGGTAATAACAACAATGGTCCGAGATCTTCAGACCTGGAGGAGGA
7100
7200

AspMetArgAspAsnTrpArgSerGluLeuTyrLysTyrLysValValLysIleGluProLeuGlyValAlaProThrLysAlaLysArgArgValValGlnArgGluLysArgAlaVal
GATATGAGGGACAATTGGAGAAGTGAATTATATAAATATAAAGTAGTAAAAATTGAACCATTAGGAGTAGCACCCACCAAGGCAAAGAGAAGAGTGGTGCAGAGAGAAAAAAGAGCAGTG
7300

GlyIleGlyAlaLeuPheLeuGlyPheLeuGlyAlaAlaGlySerThrMetGlyAlaArgSerMetThrLeuThrValGlnAlaArgGlnLeuLeuSerGlyIleValGlnGlnGlnAsn
GGAATAGGAGCTTTGTTCCTTGGGTTCTTGGGAGCAGCAGGAAGCACTATGGGCGCACGGTCAATGACGCTGACGGTACAGGCCAGACAATTATTGTCTGGTATAGTGCAGCAGCAGAAC
7400

AsnLeuLeuArgAlaIleGluAlaGlnGlnHisLeuLeuGlnLeuThrValTrpGlyIleLysGlnLeuGlnAlaArgIleLeuAlaValGluArgTyrLeuLysAspGlnGlnLeuLeu
AATTTGCTGAGGGCTATTGAGGCGCAACAGCATCTGTTGCAACTCACAGTCTGGGGCATCAAGCAGCTCCAGGCAAGAATCCTGGCTGTGGAAAGATACCTAAAGGATCAACAGCTCCTG
7500

GlyIleTrpGlyCysSerGlyLysLeuIleCysThrThrAlaValProTrpAsnAlaSerTrpSerAsnLysSerLeuGluGlnIleTrpAsnAsnMetThrTrpMetGluTrpAspArg
GGGATTTGGGGTTGCTCTGGAAAACTCATTTGCACCACTGCTGTGCCTTGGAATGCTAGTTGGAGTAATAAATCTCTGGAACAGATTTGGAATAACATGACCTGGATGGAGTGGGACAGA
7600

GluIleAsnAsnTyrThrSerLeuIleHisSerLeuIleGluGluSerGlnAsnGlnGlnGluLysAsnGlyGlnGluLeuLeuGluLeuAspLysTrpAlaSerLeuTrpAsnTrpPhe
GAAATTAACAATTACACAAGCTTAATACATTCCTTAATTGAAGAATCGCAAAACCAGCAAGAAAAGAATGAACAAGAATTATTGGAATTAGATAAATGGGCAAGTTTGTGGAATTGGTTT
7700

AsnIleThrAsnTrpLeuTrpTyrIleLysIlePheIleMetIleValGlyGlyLeuValGlyLeuArgIleValPheAlaValLeuSerIleValAsnArgValArgGlnGlyTyrSer
AACATAACAAATTGGCTGTGGTATATAAAAATATTCATAATGATAGTAGGAGGCTTGGTAGGTTTAAGAATAGTTTTTGCTGTACTTTCTATAGTGAATAGAGTTAGGCAGGGATATTCA
7800
7900

FIG. 4F

ProLeuSerPheGlnThrHisLeuProThrProArgGlyProAspArgProGluGlyIleGluGluGluGlyGlyGluArgAspArgAspArgSerIleArgLeuValAsnGlySerLeu
CCATTATCGTTTCAGACCCACCTCCCAACCCCGAGGGGACCCGACAGGCCCGAACGAATAGAAGAAGAAGGTGGAGAGAGAGACAGAGACAGATCCATTCGATTAGTGAACGGATCCTTA
                            8000

AlaLeuIleTrpAspAspLeuArgSerLeuCysLeuPheSerTyrHisArgLeuArgAspLeuLeuLeuIleValThrArgIleValGluLeuLeuGlyArgArgGlyTrpGluAlaLeu
GCACTTATCTGGGACGATCTGCGGAGCCTGTGCCTCTTCAGCTACCACCGCTTGAGAGACTTACTCTTGATTGTAACGAGGATTGTGGAACTTCTGGGACGCAGGGGGTGGGAAGCCCTC
                            8100

LysTyrTrpTrpAsnLeuLeuGlnTyrTrpSerGlnGluLeuLysAsnSerAlaValSerLeuLeuAsnAlaThrAlaIleAlaValAlaGluGlyThrAspArgValIleGluValVal
AAATATTGGTGGAATCTCCTACAGTATTGGAGTCAGCAACTAAAGAATAGTCCTGTTAGCTTCCTCAATGCCACAGCCATAGCAGTAGCTGAGCGGACAGATAGGGTTATAGAAGTAGTA
                   8200

GlnGlyAlaCysArgAlaIleArgHisIleProArgArgIleArgGlnGlyLeuGluArgIleLeuLeu *
                  ORF F — AspArgAlaTrpLysGlyPheCysTyrLys[Met]GlyGlyLysTrpSerLysSerSerValValGlyTrpProThrVal
CAAGGAGCTTGTAGAGCTATTCGCCACATACCTAGAAGAATAAGACAGGCCTTGGAAACGATTTTCCTATAAGATGGCGTGCCAAGTGGTCAAAAAGTAGTGTCGGTTGGATGGCCTACTGT
        8300                                  8400

ArgGluArgMetArgArgAlaGluProAlaAlaAspGlyValGlyAlaSerArgAspLeuGluLysHisGlyAlaIleThrSerSerAsnThrAlaAlaThrAsnAlaAlaCysAla
AAGGGAAAGAATGAGACGAGCTGAGCCAGCAGCAGATCGGGTGGCAGCAGCATCTCGAGACCTCGAAAAACATGGAGCAATCACAAGTAGCAATACAGCAGCTACCAATGCTGCTTGTCC
                            8500

TrpLeuGluAlaGlnGluGluGluGluValGlyPheProValThrProGlnValProLeuArgProMetThrTyrLysAlaAlaValAspLeuSerHisPheLeuLysGluLysGlyGly
CTGGCTAGAAGCACAAGAGGAGGAGGAGGTGCGGTTTTCCAGTCACACCTCAGGTACCTTTAAGACCAATGACTTACAAGGCAGCTGTAGATCTTAGCCACTTTTTAAAAGAAAAGCGGGGG
                            8600

LeuGluGlyLeuIleHisSerGlnArgArgGlnAspIleLeuAspLeuTrpIleTyrHisThrGlnGlyTyrPheProAspTrpGlnAsnTyrThrProGlyProGlyValArgTyrPro
ACTGGAAGGGCTAATTCACTCCCAACGAAGACAAGATATCCTTGATCTGTGGATCTACCACACACAAGGCTACTTCCCTGATTGGCAGAACTACACACCAGGGCCAGGGGTCAGATATCC
                         8700

LeuThrPheGlyTrpCysTyrLysLeuValProValGluProAspLysValGluGluAlaAsnLysGlyGluAsnThrSerLeuLeuHisProValSerLeuHisGlyMetAspAspPro
ACTGACCTTTGGATGGTGCTACAAGCTAGTACCAGTTGAGCCAGATAAGGTAGAAGAGCCCAATAAAGGAGAGAACACCAGCTTGTTACACCCTGTGAGCCTGCATGGAATGGATGACCC
                    8800

GluArgGluValLeuGluTrpArgPheAspSerArgLeuAlaPheHisHisValAlaArgGluLeuHisProGluTyrPheLysAsnCys *
TGAGAGAGAACTGTTAGAGTGGCACGTTTCGACAGCCGCCTAGCCATTTCATCACGTGGCCCCGAGAGCTGCATCCGCAGTACTTCAAGAACTGCTGACATCGAGCTTGCTACAAGGGACTTTC
        8900                                  9000

CGCTCGCGGACTTTCCAGGGAGGCGTGGCCTGGGCCCGACTGGGGAGTCGGCGAGCCCTCAGATCGTCCATATAAGCAGCTGCTTTTTGCCTGTACTGGGTCTCTCTGGTTAGACCAGATTT
                                HindIII.          9100
GACCCTGGGAGCTCTCTGGCTAACTAGGGAACCCACTGCTTAAGCCTCAATAAAGCTTGCCTTGAGTGCTTCA
                      9193

# FIG. 4G

EP 1 279 678 B1

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 8324800 A
- FR 8407151

- GB 8423659 A

### Non-patent literature cited in the description

- **F. BARRE-SINOUSSI et al.** *Science,* 1983, vol. 220, 868
- **R.C. GALLO et al.** *Science,* 1984, vol. 224, 500
- **M.G. SARNGADHARAN et al.** *Science,* 1984, vol. 224, 506
- **M. JAY LEVY et al.** *Science,* 1984, vol. 225, 840-842
- **SCHUPBACH et al.** *Science,* 04 May 1984, vol. 224, 503-505
- **DEININGER.** *Analytical Biochem.,* 1983, vol. 129, 216
- **MANIATIS T et al.** Molecular cloning. Cold Spring Harbor Laboratory, 1982
- *Gene,* 1983, vol. 26, 101
- **HANAHAN D.** *J.Mol. Biol.,* 1983, vol. 166, 557
- **SANGER et al.** *Proc. Natl. Acad. Sci. USA,* 1977, vol. 74, 5463
- **BIGGEN M.D et al.** *Proc. Natl. Acad. Sci. USA,* 1983, vol. 50, 3963
- **STADEN R.** *Nucl. Acids Res,* 1982, vol. 10, 4731
- **SCOTTO J et al.** *Hepatology,* 1983, vol. 3, 379-384
- Methoden der Organischen Chemie'' (Methods of Organic Chemistry. THIEME. 1974, vol. 15-I and
- **R.D. MERRIFIELD.** solid phase peptide synthesis. *J. Am. Chem. Soc.,* vol. 45, 2149-2154
- **FRANTZ ; ROBERTSON.** *Infect. and Immunity,* 1981, vol. 33, 193-198
- **P.E. KAUFFMAN.** *Applied and Environmental Microbiology,* October 1981, vol. 42 (4), 611-614
- **SCAND.** *J. Immunol.,* 1978, vol. 8, 7-23
- **BOQUET. P et al.** *Molec. Immunol.,* 1982, vol. 12, 1441-1549
- **ALIZON, M ; SONIGO, P ; BARRE-SINOUSSI, F ; CHERMANN, J.C ; TIOLLAIS, P ; MONTAGNIER, L ; WAIN-HOBSON, S.** Molecular cloning of lymphadenopathy-associated virus. *Nature,* 1984

- **ARYA, S.K ; GALLO, R.C ; HAHN, B.H ; SHAW, G.M ; POPOVIC, M ; SALAHUDDIN, S.Z ; WONG-STAAL, F.** Homology of genome of AIDS-associated virus with genomes of human T-cell leukemia lymphoma viruses. *Science,* 1984, vol. 225, 927-930
- **BARRÉ-SINOUSSI, F ; CHERMANN, J.C ; REY, F ; NUGEYBE, M.T ; CHAMARET, S ; GRUEST, J ; DAUGUET, C ; AXLER-BLIN, C ; VÉZINET-BRUN F ; ROUZIOUX, C.** Isolation of a T-lymphotropic retrovirus from a patient at risk of Acquired Immune Deficiency Syndrome (AIDS. *Science,* 1983, vol. 220, 868-870
- **BIGGEN, M.D ; GIBSON, T.J ; HONG, G.F.** Buffer gradient gels and S label as an aid to rapid DNA sequence determination. *Proc. Natl. Acad. Sci. USA,* 1983, vol. 80, 3963-3965
- **BIRD, A.P.** DNA methylation and the frequency of CpG in animal DNA. *Nucl. Acids Res,* 1980, vol. 8, 1499-1504
- **BRUN-VÉZINET, F ; ROUZIOUX, C ; BARRÉ-SINOUSSI, F ; KLATZMANN, D ; SAIMOT, A.G ; ROZEMBAUM, W ; MONTAGNIER, L ; CHERMANN, J.C.** Detection of IgG antibodies to lymphadenopathy associated virus (LAV) by ELISA, in patients with acquired immuno-deficiency syndrome of lymphadenopathy syndrome. *Lancet,* 1984, vol. I, 1253-1256
- **CHEN, H.R ; BARKER, W.C.** Nucleotide sequences of the retroviral long terminal repeats and their adjacent regions. *Nucl. Acids Res,* 1984, vol. 12, 1767-1773
- **CHEN, I.S.Y ; MC LAUGHLIN, J. ; GASSON, J.C ; CLARK, S.C ; GOLDE, D.W.** Molecular characterization of genome of a novel human T-cell leukaemia virus. *Nature,* 1983, vol. 303, 502-505
- **CHIU, I-M ; CALLAHAN, R ; TRONICK, S.R ; SCHOLM, J ; AARONSON, S.A.** Major pol gene progenitors in the evolution of oncornaviruses. *Science,* 1984, vol. 223, 364-370
- **CIANCIOLO, G.J ; KIPNIS, R.J ; SNYDERMAN, R.** Similarity between p15E of murine and feline viruses and p21 of HTLV. *Nature,* 1984, vol. 311, 515

- **DALY, H.M ; SCOTT, G.L.** Fatal AIDS in a haemophiliae in the U.K. *Lancet,* 1983, vol. II, 1190
- **DITTMAR, K.J ; MOELLING, K.** Biochemical properties of p15-associated protease in an avion RNA tumor virus. *J. Virol,* 1978, vol. 28, 106-113
- **DONEHOWER, L.A ; HUANG, A.L ; HAGER, G.L.** Regulatory and coding potential of the mouse mammary tumour virus long terminal redundancy. *J. Virol,* 1981, vol. 37, 226-238
- **GOTTLIEB, M.S ; SCHROFF, R ; SCHANLER, H.M ; WEISMAN, J.D ; FAN P.T ; WOLF R.A. ; SAXON, A.** Pneumocytis carinii pneumonia and mucosal candidiasis in previously healthy homosexual men : Evidence of a new acquired cellular immuno-deficiency. *N. Engl. J. Med,* 1981, vol. 305, 1426-1431
- **HAHN, B.H ; SHAW, G.M ; ARYA, S.U ; POPOVIC, M ; GALLO, R.C ; WONG-STALL, F.** Molecular cloning and characterization of the HTLV-III virus associated with AIDS. *Nature,* 1984, vol. 312, 166-169
- **HARRIS, J.D ; SCOTT, J.V ; TAYLOR, B ; BRAHIC, M ; STOWRING, L ; VENTURA, P ; HAASE, A.T ; PELUSO, R.** Visna virus DNA : discovery of a novel gapped structure. *Virology,* 1981, vol. 113, 573-583
- **KIYOKAWA; T ; YOSHIKURA, H ; HATTORI, S ; SECKI, M ; YOSHIDA, M.** Envelope proteins of human T-cell leukemia virus : expression in Escherischia coli and its application to studies of env gene functions. *Proc. Natl. Acad. Sci. USA,* 1984, vol. 81, 6202-6206
- **KLATZMANN, D ; BARRÉ-SINOUSSI, F ; NUGEYRE, M.T ; DAUGUET, C ; VILMER, E ; GRISCELLI, C ; BRUN-VÉZINET, F ; ROUZIOUXC ; GLUCKMAN, J.C ; CHERMANN, J.C.** Selective tropism of lymphadenopathy associated virus (LAV) for helper-inducer T-lymphocytes. *Sciences,* 1934, vol. 223, 59-63
- **KOZAK, M.** Compilation and analysis of sequences upstream from the transcriptional start site in eucaryotic mRNAs. *Nucl. Acids Res,* 1934, vol. 12, 857-872
- **LEVY, J.A ; HOFFMAN, A.D ; KRAMER, S.M ; LANOIS, J.A ; SHIMABUKURO, J.M ; OSKIRO, L.S.** Isolation of lymphocytopathic retroviruses from San Francisco patients with AIDS. *Science,* 1984, vol. 225, 840-842
- **MASUR, H ; MICHELIS, M.A ; GREENE, J.B ; ONOVATO, I ; VAN DE STOWE, R.A ; HOLZMAN, R.S ; WORMSER, G ; BRETTMAN, L ; LANGE, M ; MURRAY, H.W.** An outbreak of community-acquired pneumocystis carinii pneumonia : Initial manifestation of cellular immune dysfunction. *N. Engl. J. Med,* 1981, vol. 305, 1431-1438
- **MISRA, T.K ; GRANDGENETT, D.P ; PARSONS, J.T.** Avian retrovirus pp32 DNA-binding protein. I. Recognition fo specific sequences on retrovirus DNA terminal repeats. *J. Virol,* 1982, vol. 44, 330-343
- A new human T-lymphotropic retrovirus : characterization and possible role in lymphadenopathy and acquired immune deficiency syndromes. **MONTAGNIER, L et al.** In human T-cell leukemia/lymphoma viruses. Cold Spring Laboratory, 1984, 363-370
- Chemical analysis of human T-cell leukemia virus structural proteins. **OROSZIAN, S ; COPELAND, T.D ; KALYANARAMAN, V.S ; SARNGADHARAN, M.G ; SCHULTZ, A.M ; GALLO, R.C.** In HTLVS. Cold Spring Laboratory, 1984, 101-110
- **PIOT, P ; QUINN, T.C ; TAELMANN, H ; FEINSOD, F.M et al.** Acquired immunodeficiency syndrome in a heterosexual population in Zaire. *Lancet,* 1984, vol. II, 65-69
- **POPOVIC, M ; SARNGADHARAN, M.G ; READ, E ; GALLO, R.C.** Detection, isolation, and continuous production of cytopathic retroviruses (HTLV-III) from patients with AIDS and pre-AIDS. *Science,* 1984, vol. 224, 497-300
- **QUERAT, G ; BARBAN, N ; SAUZE, N ; FILIPPI, P ; VIGNE, R ; RUSSO, P ; VITU, C.** Highly lytic and persistent lentiviruses naturally present in sheep with progressive pneumonia are genetically distinct. *J. Virol,* 1984, vol. 52, 672-679
- **RABA, M ; LIMBURG, K ; BURGHAGEN, M. ; KATZE, J.R ; SIMSEK, M ; HECKMAN, J.E ; RAJBHANDARY, U.L ; GROSS, H.J.** Nucleotide sequence fo three isoaccepting lysine tRNAs from rabbit liver and SV40-transformed mouse fibroblasts. *Eur. J. Biochem.,* 1979, vol. 97, 303-318
- **RICE, N.R ; STEPHENS, R.M ; COUEZ, D ; DESCHAMPS, J ; KETTMANN, R ; BURNY, A ; GILDEN, R.V.** The nucleotide sequence of the env gene and post-env region of bovine leukemia virus. *Virology,* 1984, vol. 138, 82-93
- **SAGATA, N ; YASUNAGA, T ; OGAWA, Y ; TSUZUKU-KAWAMURA, J ; IKAWA, Y.** Bovine leukemia virus : Unique structural features of its long terminal repeats and its evolutionary relationship to human T-cell leukemia virus. *Proc. Natl. Acad. Sci. USA,* 1984, vol. 81, 4741-4745
- **SANGER, F ; NICKLEN, S ; COULSEN, A.R.** DNA sequencing with chain terminating inhibitors. *Proc. Natl. Acad. Sci. USA,* 1977, vol. 74, 5463-5467
- **SCHWARTZ, D.E. ; TIZARD, R ; GILBERT, W.** Nucleotide sequence of Rous sarcoma virus. *Cell,* 1983, vol. 32, 853-869
- **SCHÜPBACH, J ; POPOVIC, M ; GILDEN, R.V ; GONDA, M.A. ; SARNGADHARAN, M.G ; GALLO, R.C.** Serological analysis of a subgroup of human T-lymphotropic retroviruses (HTLV-III) associated with AIDS. *Science,* 1984, vol. 224, 503-505

- **SEIKI, M ; HATTORI, S ; HIRAYAMA, Y ; YOSHIDA, M.** Human adult T-cell leukemia virus : complete nucleotide sequence of the provirus genome integrated in leukemia cell DNA. *Proc. Natl. Acad. Sci. USA,* 1983, vol. 80, 3618-3622
- **SHAW, G.M ; HAHN, B.H ; ARYA, S.K ; GROOPMAN, J.E ; GALLO, R.C ; WONG-STAAL, F.** Molecular characterization of human T-cell leukemia (lymphotropic) virus type III in the Acquired Immune Deficiency Syndrome. *Science,* 1984, vol. 226, 1163-1171
- **SHIMOTOHNO, K ; TEMIN, H.M.** Spontaneous variation and synthesis in the U3 region of the long terminal repeat of avion retroviruses. *J. Virol.,* 1982, vol. 41, 1636171
- **SHIMOTOHNO, K ; GOLDE, D.M ; MIWA, M ; SUGIMURA, T ; CHEN, I.S.Y.** Nucleotide sequence analysis to the long terminal repeat of human T-cell leukemia virus type II. *Proc. Natl. Acad. Sci. USA,* 1984, vol. 81, 1079-1083
- **SHINNICK, T.M ; LERNER, R.A ; SUTCLIFFE, J.C.** Nucleotide sequence of Moloney murine leukemia viruse. *Nature,* 1981, vol. 293, 543-548
- **SRINIVASAN, A ; REDDY, E.P ; DUNN, C.Y ; AARONSON, S.A.** Molecular dissection of transcriptional control elements with the long terminal repeat of retrovirus. *Science,* 1984, vol. 223, 286-289